(19) Europäisches Patentamt
European Patent Office
Office européen des brevets

(11) EP 2 635 676 B1

(12) **EUROPEAN PATENT SPECIFICATION**

(45) Date of publication and mention of the grant of the patent:
**22.04.2015 Bulletin 2015/17**

(21) Application number: **11778880.2**

(22) Date of filing: **03.11.2011**

(51) Int Cl.:
***C12N 9/36*** (2006.01) ***C12N 9/52*** (2006.01)
***A61K 38/46*** (2006.01)

(86) International application number:
**PCT/EP2011/069336**

(87) International publication number:
**WO 2012/059545 (10.05.2012 Gazette 2012/19)**

(54) **NOVEL ENDOLYSIN**

NEUES ENDOLYSIN

NOUVELLE ENDOLYSINE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **03.11.2010 GB 201018518**

(43) Date of publication of application:
**11.09.2013 Bulletin 2013/37**

(73) Proprietors:
- **Katholieke Universiteit Leuven, K.U. Leuven R&D 3000 Leuven (BE)**
- **University Of Minho 4704-553 Braga (PT)**

(72) Inventors:
- **RODRIGUES AZEREDO, Joana Cecilia Valente 4700-752 Braga (PT)**
- **BRANCO DOS SANTOS, Silvio Roberto 4575-379 Pinheiro-Penafiel (PT)**
- **KLUSKENS, Leonardus Dorothea 4730-573 Braga (PT)**
- **LAVIGNE, Rob 2180 Ekeren (BE)**
- **WALMAGH, Maarten 3540 Herk-de-Stad (BE)**

(74) Representative: **Rückerl, Florian Dehmel und Bettenhausen Herzogspitalstraße 11 80331 München (DE)**

(56) References cited:
**WO-A2-2010/023207**

- **SANTOS S B ET AL: "Selection and characterization of a multivalent Salmonella phage and its production in a nonpathogenic Escherichia coli strain", APPLIED AND ENVIRONMENTAL MICROBIOLOGY, AMERICAN SOCIETY FOR MICROBIOLOGY, US, vol. 76, no. 21, 3 September 2010 (2010-09-03), pages 7338-7342, XP009146098, ISSN: 0099-2240, DOI: 10.1128/AEM.00922-10**
- **ORITO Y ET AL: "Bacillus amyloliquefaciens phage endolysin can enhance permeability of Pseudomonas aeruginosa outer membrane and induce cell lysis", APPLIED MICROBIOLOGY AND BIOTECHNOLOGY, SPRINGER VERLAG, BERLIN, DE, vol. 65, no. 1, 1 July 2004 (2004-07-01), pages 105-109, XP002579253, ISSN: 0175-7598, DOI: 10.1007/S00253-003-1522-1 [retrieved on 2004-01-09]**
- **ARIMA H ET AL: "Bactericidal action of lysozymes attached with various sizes of hydrophobic peptides to the C-terminal using genetic modification", FEBS LETTERS, ELSEVIER, AMSTERDAM, NL, vol. 415, no. 1, 22 September 1997 (1997-09-22), pages 114-118, XP004261147, ISSN: 0014-5793, DOI: 10.1016/S0014-5793(97)01071-5**
- **MAYER MELINDA J ET AL: "Molecular characterization of a Clostridium difficile bacteriophage and its cloned biologically active endolysin", JOURNAL OF BACTERIOLOGY, AMERICAN SOCIETY FOR MICROBIOLOGY, WASHINGTON, DC; US, vol. 190, no. 20, 1 October 2008 (2008-10-01), pages 6734-6740, XP002597086, ISSN: 0021-9193, DOI: 10.1128/JB.00686-08**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **AMORIM L R P ET AL: "Numeric taxonomy approaches for lytic evaluation of Salmonella specific bacteriophages", FOOD CONTROL, BUTTERWORTH, LONDON, GB, vol. 21, no. 5, 1 May 2010 (2010-05-01), pages 754-759, XP026835986, ISSN: 0956-7135 [retrieved on 2009-11-14]**

SEQUENCE LISTING

<110> Katholieke Universiteit Leuven K.U.Leuven R&D University of Minho

<120> NOVEL ENDOLYSIN

<130> LYS-014 PCT

<140> not yet known
<141> 2011-11-03

<150> GB 1018518.9
<151> 2010-11-03

<160> 31

<170> PatentIn version 3.3

<210> 1
<211> 235
<212> PRT
<213> Artificial Sequence

<220>
<223> wt PVPSE1gp146

<400> 1

```
Met Asn Ala Ala Ile Ala Glu Ile Gln Arg Met Leu Ile Glu Gly Gly
1               5                   10                  15

Phe Ser Val Gly Lys Ser Gly Ala Asp Gly Leu Tyr Gly Pro Ala Thr
            20                  25                  30

Lys Ala Ala Leu Gln Lys Cys Ile Ala Gln Ala Thr Ser Gly Asn Asn
        35                  40                  45

Lys Gly Gly Thr Leu Lys Leu Thr Gln Ala Gln Leu Asp Lys Ile Phe
    50                  55                  60

Pro Val Gly Ala Ser Ser Gly Arg Asn Ala Lys Leu Lys Pro Leu Asn
65                  70                  75                  80

Asp Leu Phe Glu Lys Thr Glu Ile Asn Thr Val Asn Arg Val Ala Gly
                85                  90                  95

Phe Leu Ser Gln Ile Gly Val Glu Ser Ala Glu Phe Arg Tyr Val Arg
            100                 105                 110

Glu Leu Gly Asn Asp Ala Tyr Phe Asp Lys Tyr Asp Thr Gly Pro Ile
        115                 120                 125

Ala Glu Arg Leu Gly Asn Thr Pro Gln Lys Asp Gly Asp Gly Ala Lys
    130                 135                 140

Tyr Lys Gly Arg Gly Leu Ile Gln Val Thr Gly Leu Ala Asn Tyr Lys
145                 150                 155                 160

Ala Cys Gly Lys Ala Leu Gly Leu Asp Leu Val Asn His Pro Glu Leu
                165                 170                 175

Leu Glu Gln Pro Glu Tyr Ala Val Ala Ser Ala Gly Trp Tyr Trp Asp
            180                 185                 190

Thr Arg Asn Ile Asn Ala Ala Cys Asp Ala Asp Asp Ile Val Lys Ile
        195                 200                 205

Thr Lys Leu Val Asn Gly Gly Thr Asn His Leu Ala Glu Arg Thr Ala
    210                 215                 220

Tyr Tyr Lys Lys Ala Lys Ser Val Leu Thr Ser
225                 230                 235
```

<210> 2
<211> 711
<212> DNA

<213> Artificial Sequence

<220>
<223> wt ORF146 of PVPSE1

<400> 2

```
atgaatgctg caattgcgga gattcagcgt atgctgatcg aaggtgggtt tagcgtcggc     60
aagtctggtg ctgatggatt gtacggaccc gctacaaaag ccgcactgca aaagtgcatt    120
gcacaggcta ccagtggaaa caataaagga ggtactttga aactcaccca agcacaactg    180
gacaaaatct tccccgttgg tgcaagttct gggaggaatg caaaattcct gaagccgctc    240
aatgacctgt ttgaaaagac agagattaat acggtaaatc gggttgcagg attcctgtct    300
cagattggtg tggagtcggc ggagttccgg tatgtacgtg aactcggtaa cgatgcctac    360
tttgacaagt acgacactgg tcctattgca gaaagacttg gaaacacacc ccagaaagat    420
ggggatggtg ccaagtacaa ggggagaggt ctgattcagg tgaccggact cgcaaactac    480
aaggcttgcg gtaaagcact cggtcttgac ctcgttaacc accctgagtt gcttgaacag    540
cctgagtatg cagttgccag cgctggttgg tattgggaca cgagaaacat caacgccgct    600
tgcgatgctg atgatatcgt gaaaattacc aagctggtaa acggtggtac aaatcacctt    660
gccgagcgca cagcctatta caaaaaggca aaatctgttc taacctcgta a             711
```

<210> 3
<211> 37
<212> PRT
<213> Artificial Sequence

<220>
<223> peptidoglycan binding domain of PVPSE1

<400> 3

```
Ala Ala Ile Ala Glu Ile Gln Arg Met Leu Ile Glu Gly Gly Phe Ser
1               5                   10                  15

Val Gly Lys Ser Gly Ala Asp Gly Leu Tyr Gly Pro Ala Thr Lys Ala
            20                  25                  30

Ala Leu Gln Lys Cys
        35
```

<210> 4
<211> 111
<212> DNA
<213> Artificial sequence

<220>
<223> pepdidoglycan binding domain of PVPSE1

<400> 4

gctgcaattg cggagattca gcgtatgctg atcgaaggtg ggtttagcgt cggcaagtct 60

ggtgctgatg gattgtacgg acccgctaca aaagccgcac tgcaaaagtg c 111

<210> 5
<211> 153
<212> PRT
<213> Artificial Sequence

<220>
<223> catalytic domain of PVPSE1

<400> 5

Asp Leu Phe Glu Lys Thr Glu Ile Asn Thr Val Asn Arg Val Ala Gly
1 5 10 15

Phe Leu Ser Gln Ile Gly Val Glu Ser Ala Glu Phe Arg Tyr Val Arg
20 25 30

Glu Leu Gly Asn Asp Ala Tyr Phe Asp Lys Tyr Asp Thr Gly Pro Ile
35 40 45

Ala Glu Arg Leu Gly Asn Thr Pro Gln Lys Asp Gly Asp Gly Ala Lys
50 55 60

Tyr Lys Gly Arg Gly Leu Ile Gln Val Thr Gly Leu Ala Asn Tyr Lys
65 70 75 80

Ala Cys Gly Lys Ala Leu Gly Leu Asp Leu Val Asn His Pro Glu Leu
85 90 95

Leu Glu Gln Pro Glu Tyr Ala Val Ala Ser Ala Gly Trp Tyr Trp Asp
100 105 110

Thr Arg Asn Ile Asn Ala Ala Cys Asp Ala Asp Asp Ile Val Lys Ile
115 120 125

Thr Lys Leu Val Asn Gly Gly Thr Asn His Leu Ala Glu Arg Thr Ala
130 135 140

Tyr Tyr Lys Lys Ala Lys Ser Val Leu
145 150

<210> 6
<211> 459
<212> DNA
<213> Artificial Sequence

<220>
<223> catalytic domain of PVPSE1

<400> 6

```
gacctgtttg aaaagacaga gattaatacg gtaaatcggg ttgcaggatt cctgtctcag     60
attggtgtgg agtcggcgga gttccggtat gtacgtgaac tcggtaacga tgcctacttt    120
gacaagtacg acactggtcc tattgcagaa agacttggaa acacacccca gaaagatggg    180
gatggtgcca agtacaaggg gagaggtctg attcaggtga ccggactcgc aaactacaag    240
gcttgcggta aagcactcgg tcttgacctc gttaaccacc ctgagttgct tgaacagcct    300
gagtatgcag ttgccagcgc tggttggtat tgggacacga gaaacatcaa cgccgcttgc    360
gatgctgatg atatcgtgaa aattaccaag ctggtaaacg gtggtacaaa tcaccttgcc    420
gagcgcacag cctattacaa aaaggcaaaa tctgttcta                           459
```

<210> 7
<211> 245
<212> PRT
<213> Artificial Sequence

<220>
<223> recombinant PVPSE1gp146

<400> 7

```
Met Gly Ser Asn Ala Ala Ile Ala Glu Ile Gln Arg Met Leu Ile Glu
1               5                   10                  15

Gly Gly Phe Ser Val Gly Lys Ser Gly Ala Asp Gly Leu Tyr Gly Pro
            20                  25                  30
```

Ala Thr Lys Ala Ala Leu Gln Lys Cys Ile Ala Gln Ala Thr Ser Gly
35 40 45

Asn Asn Lys Gly Gly Thr Leu Lys Leu Thr Gln Ala Gln Leu Asp Lys
50 55 60

Ile Phe Pro Val Gly Ala Ser Ser Gly Arg Asn Ala Lys Leu Lys Pro
65 70 75 80

Leu Asn Asp Leu Phe Glu Lys Thr Glu Ile Asn Thr Val Asn Arg Val
85 90 95

Ala Gly Phe Leu Ser Gln Ile Gly Val Glu Ser Ala Glu Phe Arg Tyr
100 105 110

Val Arg Glu Leu Gly Asn Asp Ala Tyr Phe Asp Lys Tyr Asp Thr Gly
115 120 125

Pro Ile Ala Glu Arg Leu Gly Asn Thr Pro Gln Lys Asp Gly Asp Gly
130 135 140

Ala Lys Tyr Lys Gly Arg Gly Leu Ile Gln Val Thr Gly Leu Ala Asn
145 150 155 160

Tyr Lys Ala Cys Gly Lys Ala Leu Gly Leu Asp Leu Val Asn His Pro
165 170 175

Glu Leu Leu Glu Gln Pro Glu Tyr Ala Val Ala Ser Ala Gly Trp Tyr
180 185 190

Trp Asp Thr Arg Asn Ile Asn Ala Ala Cys Asp Ala Asp Asp Ile Val
195 200 205

Lys Ile Thr Lys Leu Val Asn Gly Gly Thr Asn His Leu Ala Glu Arg
210 215 220

Thr Ala Tyr Tyr Lys Lys Ala Lys Ser Val Leu Thr Ser Lys Gly His
225 230 235 240

His His His His His
245

<210> 8
<211> 741
<212> DNA
<213> Artificial Sequence

<220>

<223> recombinant PVPSE1gp146

<400> 8

```
atgggatcca atgctgcaat tgcggagatt cagcgtatgc tgatcgaagg tgggtttagc      60
gtcggcaagt ctggtgctga tggattgtac ggacccgcta caaaagccgc actgcaaaag     120
tgcattgcac aggctaccag tggaaacaat aaaggaggta ctttgaaact cacccaagca     180
caactggaca aaatcttccc cgttggtgca agttctggga ggaatgcaaa attcctgaag     240
ccgctcaatg acctgtttga aaagacagag attaatacgg taaatcgggt tgcaggattc     300
ctgtctcaga ttggtgtgga gtcggcggag ttccggtatg tacgtgaact cggtaacgat     360
gcctactttg acaagtacga cactggtcct attgcagaaa gacttggaaa cacaccccag     420
aaagatgggg atggtgccaa gtacaagggg agaggtctga ttcaggtgac cggactcgca     480
aactacaagg cttgcggtaa agcactcggt cttgacctcg ttaaccaccc tgagttgctt     540
gaacagcctg agtatgcagt tgccagcgct ggttggtatt gggacacgag aaacatcaac     600
gccgcttgcg atgctgatga tatcgtgaaa attaccaagc tggtaaacgg tggtacaaat     660
caccttgccg agcgcacagc ctattacaaa aaggcaaaat ctgttctaac ctcgaagggt     720
catcatcacc atcaccattg a                                               741
```

<210> 9
<211> 28
<212> DNA
<213> Artificial Sequence

<220>
<223> ORF146 forward primer

<400> 9
atgggatcca atgctgcaat tgcggaga 28

<210> 10
<211> 23
<212> DNA
<213> Artificial Sequence

<220>
<223> ORF146 reverse primer

<400> 10
cgaggttaga acagattttg cct 23

<210> 11
<211> 55
<212> DNA
<213> Artificial Sequence

<220>
<223> extended ORF146 forward primer

<400> 11
atgggatcca aacgcaagaa acgtaagaaa cgcaaaaatg ctgcaattgc ggaga 55

<210> 12
<211> 768
<212> DNA
<213> Artificial Sequence

<220>
<223> DNA sequence of recombinant PK-PVPSE1gp146

<400> 12

```
atgggatcca aacgcaagaa acgtaagaaa cgcaaaaatg ctgcaattgc ggagattcag     60
cgtatgctga tcgaaggtgg gtttagcgtc ggcaagtctg gtgctgatgg attgtacgga    120
cccgctacaa aagccgcact gcaaaagtgc attgcacagg ctaccagtgg aaacaataaa    180
ggaggtactt tgaaactcac ccaagcacaa ctggacaaaa tcttccccgt tggtgcaagt    240
tctgggagga atgcaaaatt cctgaagccg ctcaatgacc tgtttgaaaa gacagagatt    300
aatacggtaa atcgggttgc aggattcctg tctcagattg gtgtggagtc ggcggagttc    360
cggtatgtac gtgaactcgg taacgatgcc tactttgaca agtacgacac tggtcctatt    420
gcagaaagac ttggaaacac accccagaaa gatggggatg gtgccaagta caaggggaga    480
ggtctgattc aggtgaccgg actcgcaaac tacaaggctt gcggtaaagc actcggtctt    540
gacctcgtta accaccctga gttgcttgaa cagcctgagt atgcagttgc cagcgctggt    600
tggtattggg acacgagaaa catcaacgcc gcttgcgatg ctgatgatat cgtgaaaatt    660
accaagctgg taaacggtgg tacaaatcac cttgccgagc gcacagccta ttacaaaaag    720
gcaaaatctg ttctaacctc gaagggtcat catcaccatc accattga                 768
```

<210> 13
<211> 254
<212> PRT
<213> Artificial Sequence

<220>
<223> amino acid sequence of recombinant PK-PVPSE1gp146

<400> 13

```
Met Gly Ser Lys Arg Lys Lys Arg Lys Lys Arg Lys Asn Ala Ala Ile
1               5                   10                  15

Ala Glu Ile Gln Arg Met Leu Ile Glu Gly Gly Phe Ser Val Gly Lys
            20                  25                  30

Ser Gly Ala Asp Gly Leu Tyr Gly Pro Ala Thr Lys Ala Ala Leu Gln
        35                  40                  45

Lys Cys Ile Ala Gln Ala Thr Ser Gly Asn Asn Lys Gly Gly Thr Leu
    50                  55                  60

Lys Leu Thr Gln Ala Gln Leu Asp Lys Ile Phe Pro Val Gly Ala Ser
65                  70                  75                  80

Ser Gly Arg Asn Ala Lys Leu Lys Pro Leu Asn Asp Leu Phe Glu Lys
                85                  90                  95

Thr Glu Ile Asn Thr Val Asn Arg Val Ala Gly Phe Leu Ser Gln Ile
            100                 105                 110

Gly Val Glu Ser Ala Glu Phe Arg Tyr Val Arg Glu Leu Gly Asn Asp
        115                 120                 125

Ala Tyr Phe Asp Lys Tyr Asp Thr Gly Pro Ile Ala Glu Arg Leu Gly
    130                 135                 140

Asn Thr Pro Gln Lys Asp Gly Asp Gly Ala Lys Tyr Lys Gly Arg Gly
145                 150                 155                 160

Leu Ile Gln Val Thr Gly Leu Ala Asn Tyr Lys Ala Cys Gly Lys Ala
                165                 170                 175

Leu Gly Leu Asp Leu Val Asn His Pro Glu Leu Leu Glu Gln Pro Glu
            180                 185                 190

Tyr Ala Val Ala Ser Ala Gly Trp Tyr Trp Asp Thr Arg Asn Ile Asn
        195                 200                 205

Ala Ala Cys Asp Ala Asp Asp Ile Val Lys Ile Thr Lys Leu Val Asn
    210                 215                 220

Gly Gly Thr Asn His Leu Ala Glu Arg Thr Ala Tyr Tyr Lys Lys Ala
225                 230                 235                 240

Lys Ser Val Leu Thr Ser Lys Gly His His His His His His
                245                 250
```

<210> 14
<211> 13
<212> PRT
<213> Artificial Sequence

<220>
<223> alpha4-helix of T4-lysozyme

<400> 14

```
Pro Asn Arg Ala Lys Arg Val Ile Thr Thr Phe Arg Thr
1               5                   10
```

<210> 15
<211> 5
<212> PRT
<213> Artificial Sequence

<220>
<223> Pentapeptide

<400> 15

```
Phe Phe Val Ala Pro
1               5
```

<210> 16
<211> 18
<212> PRT
<213> Artificial Sequence

<220>
<223> Artilysin 1

<400> 16

```
Gly Phe Phe Ile Pro Ala Val Ile Leu Pro Ser Ile Ala Phe Leu Ile
1               5                   10                  15

Val Pro
```

<210> 17
<211> 25
<212> PRT
<213> Artificial Sequence

<220>
<223> Artilysin 2

<400> 17

```
Gly Lys Pro Gly Trp Leu Ile Lys Lys Ala Leu Val Phe Lys Lys Leu
1               5                   10                  15

Ile Arg Arg Pro Leu Lys Arg Leu Ala
            20                  25
```

<210> 18
<211> 19
<212> PRT
<213> Artificial Sequence

<220>
<223> Parasin 1

<400> 18

```
Lys Gly Arg Gly Lys Gln Gly Gly Lys Val Arg Ala Lys Ala Lys Thr
1               5                   10                  15

Arg Ser Ser
```

<210> 19
<211> 25
<212> PRT
<213> Artificial Sequence

<220>
<223> Lycotoxin 1

<400> 19

```
Ile Trp Leu Thr Ala Leu Lys Phe Leu Gly Lys His Ala Ala Lys Lys
1               5                   10                  15

Leu Ala Lys Gln Gln Leu Ser Lys Leu
            20                  25
```

<210> 20
<211> 39
<212> DNA
<213> Artificial Sequence

<220>
<223> forward primer of alpha4-helix of T4-lysozyme

<400> 20
ttggaatggg gagcccgaac cgtgcaaaac gtgtaatca 39

<210> 21
<211> 39
<212> DNA
<213> Artificial Sequence

<220>
<223> reverse primer of alpha4-helix of T4-lysozyme

<400> 21
cattggagga gccggtacgg aaggtggtga ttacacgtt 39

<210> 22
<211> 39
<212> DNA

<213> Artificial Sequence

<220>
<223> forward primer of Artilysin 1

<400> 22
ttatgggctt cttcatcccg gcagtaatcc tgccctcca 39

<210> 23
<211> 46
<212> DNA
<213> Artificial Sequence

<220>
<223> reverse primer of Artilysin 1

<400> 23
cattggagga gcccggtacg atcaggaatg cgatggaggg caggat 46

<210> 24
<211> 47
<212> DNA
<213> Artificial Sequence

<220>
<223> forward primer of Artilysin 2

<400> 24
ttatgggcaa accgggctgg ctgatcaaaa ggcactggta ttcaaga 47

<210> 25
<211> 57
<212> DNA
<213> Artificial Sequence

<220>
<223> reverse primer of Artilysin 2

<400> 25
cattggagga gcctgccagt ctcttcagcg gacgacggat cagtttcttg aatacca 57

<210> 26
<211> 48
<212> DNA
<213> Artificial Sequence

<220>
<223> forward primer of Parasin 1

<400> 26
ttggaatggg gagcaaaggc cgtggcaagc agggaggcaa agtacgtg 48

<210> 27
<211> 48
<212> DNA
<213> Artificial Sequence

<220>

<223> reverse primer of Parasin 1

<400> 27
cattggagga gcctgaggaa cgggtctttg cttttgcacg tactttgc 48

<210> 28
<211> 55
<212> DNA
<213> Artificial Sequence

<220>
<223> forward primer of Lycotoxin 1

<400> 28
ggaatgggga gcatctggct gaccgcactg aaattcctcg gcaaacacgc cgcaa 55

<210> 29
<211> 57
<212> DNA
<213> Artificial Sequence

<220>
<223> reverse primer of Lycotoxin 1

<400> 29
cattggagga gcccagtttg gataattgct gttttgccag tttctttgcg gcgtgtt 57

<210> 30
<211> 38
<212> DNA
<213> Artificial Sequence

<220>
<223> 5’ forward primer for tail PCR

<400> 30
ggaatgggga gctcctccaa tgctgcaatt gcggagat 38

<210> 31
<211> 44
<212> DNA
<213> Artificial Sequence

<220>
<223> extended 5’ forward primer for pentapeptide

<400> 31
atgggatcct tcttcgtagc accgggctcc tccaatgctg caat 44

## Description

### FIELD OF THE INVENTION

The present invention relates to a polypeptide having endolysin activity with an amino acid sequence according to SEQ ID NO: 1 and fragments or derivatives thereof as set forth in claim 1. Moreover, the present invention relates to nucleic acid molecules encoding said polypeptide or fragment or derivatives thereof, vectors comprising said nucleic acid molecules and host cells comprising either said nucleic acid molecules or said vectors. In addition, the present invention relates to said polypeptide, fragments or derivatives thereof for use as a medicament, in particular for the treatment or prevention of Gram-negative bacterial infections, as diagnostic means, as cosmetic substance or as sanitizing agent. The present invention also relates to the use of said polypeptide, fragments or derivatives thereof for the treatment or prevention of bacterial contamination, particularly of Gram-negative contamination, of foodstuff, of food processing equipment, of food processing plants, of surfaces coming into contact with foodstuff, of medical devices, of surfaces in hospitals and surgeries. Furthermore, the present invention relates to a pharmaceutical composition comprising said polypeptide, fragments or derivatives thereof.

### BACKGROUND OF THE INVENTION

Gram-negative bacteria possess an outer membrane with its characteristic asymmetric bilayer as a hallmark. The outer membrane bilayer consists of an inner monolayer containing phospholipids (primarily phosphatidyl ethanolamine) and an outer monolayer that is mainly composed of a single glycolipid, lipopolysaccharide (LPS). There is an immense diversity of LPS structures in the bacterial kingdom and the LPS structure may be modified in response to prevailing environmental conditions. The stability of the LPS layer and interaction between different LPS molecules is mainly achieved by the electrostatic interaction of divalent ions ($Mg^{2+}$, $Ca^{2+}$) with the anionic components of the LPS molecule (phosphate groups in the lipid A and the inner core and carboxyl groups of KDO). Furthermore, the dense and ordered packing of the hydrophobic moiety of lipid A, favored by the absence of unsaturated fatty acids, forms a rigid structure with high viscosity. This makes it less permeable for lipophilic molecules and confers additional stability to the outer membrane (OM).

Various types of agents having bactericidal or bacteriostatic activity are known, e.g. antibiotics, endolysins, antimicrobial peptides and defensins. Increasingly microbial resistance to antibiotics, however, is creating difficulties in treating more and more infections caused by bacteria. Particular difficulties arise with infections caused by Gram-negative bacteria like Enterobacteriaceae, such as *Salmonella sp.,* and *Pseudomonas aeruginosa*.

Endolysins are peptidoglycan hydrolases encoded by bacteriophages (or bacterial viruses). They are synthesized during late gene expression in the lytic cycle of phage multiplication and mediate the release of progeny virions from infected cells through degradation of the bacterial peptidoglycan. They are either β(1,4)-glycosylases (lysozymes), transglycosylases, amidases or endopeptidases. Antimicrobial application of endolysins was already suggested in 1991 by Gasson (GB2243611). Although the killing capacity of endolysins has been known for a long time, the use of these enzymes as antibacterials was ignored due to the success and dominance of antibiotics. Only after the appearance of multiple antibiotic resistant bacteria this simple concept of combating human pathogens with endolysins received interest. A compelling need to develop totally new classes of antibacterial agents emerged and endolysins used as 'enzybiotics' - a hybrid term of 'enzymes' and 'antibiotics' - perfectly met this need. In 2001, Fischetti and coworkers demonstrated for the first time the therapeutic potential of bacteriophage Cl endolysin towards group A streptococci (Nelson et al., 2001). Since then many publications have established endolysins as an attractive and complementary alternative to control bacterial infections, particularly by Gram positive bacteria. Subsequently different endolysins against other Gram positive pathogens such as *Streptococcus pneumoniae* (Loeffler et al., 2001), *Bacillus anthracis* (Schuch et al., 2002), S. *agalactiae* (Cheng et al., 2005) and *Staphylococcus aureus* (Rashel et al, 2007) have proven their efficacy as enzybiotics. Nowadays, the most important challenge of endolysin therapy lies in the insensitivity of Gram-negative bacteria towards the exogenous action of endolysins, since the outer membrane shields the access of endolysins from the peptidoglycan. This currently prevents the expansion of the range of effective endolysins to important Gram-negative pathogens.

Antimicrobial peptides (AMPs) represent a wide range of short, cationic or amphipatic, gene encoded peptide antibiotics that can be found in virtually every organism. Different AMPs display different properties, and many peptides in this class are being intensively researched not only as antibiotics, but also as templates for cell penetrating peptides. Despite sharing a few common features (e.g., cationicity, amphipathicity and short size), AMP sequences vary greatly, and at least four structural groups (α-helical, β-sheet, extended and looped) have been proposed to accommodate the diversity of the observed AMP conformations. Likewise, several modes of action as antibiotics have been proposed, and it was shown e.g. that the primary target of many of these peptides is the cell membrane whereas for other peptides the primary target is cytoplasmic invasion and disruption of core metabolic functions. AMPs may become concentrated

enough to exhibit cooperative activity despite the absence of specific target binding; for example, by forming a pore in the membrane, as is the case for most AMPs. However, this phenomenon has only been observed in model phospholipid bilayers, and in some cases, AMP concentrations in the membrane that were as high as one peptide molecule per six phospholipid molecules were required for these events to occur. These concentrations are close to, if not at, full membrane saturation. As the minimum inhibitory concentration (MIC) for AMPs are typically in the low micromolar range, scepticism has understandably arisen regarding the relevance of these thresholds and their importance *in vivo* (Melo et al., Nature reviews, Microbiology, 2009, 245).

Defensins are a large family of small, cationic, cysteine- and arginine-rich antimicrobial peptides, found in both vertebrates and invertebrates. Defensins are divided into five groups according to the spacing pattern of cysteines: plant, invertebrate, α-, β-, and θ-defensins. The latter three are mostly found in mammals. α-defensins are proteins found in neutrophils and intestinal epithelia. β-defensins are the most widely distributed and are secreted by leukocytes and epithelial cells of many kinds. θ-defensins have been rarely found so far e.g. in leukocytes of rhesus macaques. Defensins are active against bacteria, fungi and many enveloped and nonenveloped viruses. However, the concentrations needed for efficient killing of bacteria are mostly high, i.e. in the micromolar range. Activity of many peptides may be limited in presence of physiological salt conditions, divalent cations and serum. Depending on the content of hydrophobic amino acid residues defensins also show haemolytic activity.

WO 2010023207 relates to endolysin variants comprising an endolysin to which a peptide stretch with membrane or LPS disrupting activity is fused and their use as a medicament, in particular for the treatment or prevention of Gram-negative bacterial infections, as diagnostic means, disinfectant or as cosmetic substance.

Santos et al. (Appl Environ Microbiol. 2010 Nov;76(21):7338-42) report the selection and amplification of the broad-host-range Salmonella phage phi PVP-SE1 in an alternative nonpathogenic host.

Thus, there is a need for new antimicrobial agents against Gram-negative bacteria.

## SUMMARY OF THE INVENTION

The present invention relates to a novel polypeptide with endolysin activity, isolated from *Salmonella enteritis* phage PVPSE1 and which is useful in the preparation of novel antibacterial agents against Gram-negative bacteria.

A first object of the present invention provides a polypeptide having endolysin activity comprising the amino acid sequence according to SEQ ID NO: 1 or a fragment or derivative thereof, wherein the fragment comprises the amino acid sequence according to SEQ ID NO: 3 and/or 5, and the derivative has a deletion of 1, 2, 3, 4, 5 or more amino acid residues, an addition of 1, 2, 3, 4, 5 or more amino acid residues, an insertion of 1, 2, 3, 4, 5 or more amino acid residues, or a substitution of an amino acid residue located at a certain position for a different one in the amino acid sequence according to SEQ ID NO: 1, 3, and/or 5, and wherein the fragment and/or the derivative exhibit the lytic activity of the PVPSE1gp146 set forth in SEQ ID NO: 1.

The polypeptide comprising an amino acid sequence according to SEQ ID NO:1 is preferably encoded by a nucleotide sequence according to SEQ ID NO:2.

In a preferred embodiment said fragment comprising the amino acid sequence according to SEQ ID NO: 3 and/or 5 is encoded by nucleotide sequence SEQ ID NO: 4 and/or SEQ ID NO: 6, respectively.

In yet another preferred embodiment, said polypeptide according to the present invention or a fragment or derivative thereof is fused at the N- or C-terminus to a peptide stretch having membrane or LPS disrupting activity, in particular a cationic or polycationic peptide.

Said polypeptide, fragment or derivative thereof or fusion protein may additionally comprise a tag, preferably a His6-tag.

In a preferred embodiment of the present invention, the polypeptide comprises an amino acid sequence according to SEQ ID NO:7.

A further object of the present invention relates to a fusion protein comprising the aforementioned polypeptide and a peptide stretch fused to said polypeptide at the N- or C-terminus, wherein said peptide stretch is a cationic peptide, polycationic peptide, amphipathic peptide, sushi peptide, defensin, hydrophobic peptide and/or an antimicrobial peptide.

In a preferred embodiment of the present invention, said peptide stretch comprises 5 to 100 amino acid residues, in particular 5 to 50 amino acid residues, in particular 5 to 30 amino acid residues.

In a further preferred embodiment of the present invention said cationic and/or polycationic peptide stretch comprises at least one amino acid residue selected out of the group consisting of arginine, histidine and lysine residues, in particular wherein at least 70% of the amino acid residues comprised in said peptide stretch are arginine, histidine and/or lysine residues, in particular arginine, and/or lysine residues.

In a preferred embodiment of the present invention the amphipathic peptide comprises at least one positively charged amino acid residues selected out of the group consisting of lysine, arginine and histidine residues, combined to at least one hydrophobic amino acid residue selected out of the group consisting of valine, isoleucine, leucine, methionine, phenylalanine, tryptophan, cysteine, alanine, tyrosine, histidine, threonin, serine, proline and glycine residues, in particular wherein at least 70% of the said amino acid residues in said amphipathic peptide are either arginine or lysine residues and at least 30% of the said amino acid residues in said amphipathic peptide are valine, isoleucine, leucine, methionine, phenylalanine, tryptophan, cysteine, alanine, tyrosine, histidine, threonine, serine, proline or glycine residues.

In a further preferred embodiment of the present invention comprises the peptide stretch an amino acid sequence according to SEQ ID NO:14-19.

In a particular preferred embodiment of the present invention comprises the fusion protein an amino acid sequence according to SEQ ID NO: 13.

A further object of the present invention relates to a vector comprising the nucleic acid molecule according to claim 13.

A further object of the present invention provides an isolated nucleic acid molecule encoding a polypeptide, a fragment or derivative thereof or a fusion protein according to the invention.

A further object of the present invention relates to a vector comprising a nucleic acid molecule according to the present invention.

A further object of the present invention relates to a host cell comprising a nucleic acid molecule according to the present invention or a vector according to the present invention.

A further object of the present invention relates to the polypeptide, fragment or derivative or according to the present invention or to the fusion protein according to the present invention for use in a method of treatment of the human or animal body by surgery or therapy or for use in diagnostic methods practised on the human or animal body, or for use as an antimicrobial in food or on cosmetics, as disinfecting agent or in the environmental field.

In a preferred embodiment, said polypeptide, a fragment or derivative or a fusion protein according to the present invention is used in a method for treatment or prevention of Gram-negative bacterial infections.

Another preferred embodiment relates to the use of said polypeptide, a fragment or derivative or the fusion protein according to the present invention for the treatment or prevention of Gram-negative bacterial contamination of foodstuff, of food processing equipment, of food processing plants, of shelve surfaces coming into contact with foodstuff, of medical devices, of surfaces in hospitals and surgeries.

A further preferred embodiment of the present invention relates to a pharmaceutical composition comprising the polypeptide of the present invention or the fusion protein of the present invention.

## DETAILED DESCRIPTION

### Legends to the figures

The following figures serve to illustrate the invention.

Figure 1 shows the functional analysis of the wild type modular *Salmonella enteritis* endolysin PVPSE1gp146 using BLASTp and Pfam analysis systems. The amino acid and DNA and amino acid sequences as they appear in the wild type phage are SEQ ID No. 1 and SEQ ID No. 2, respectively. The predicted N-terminal peptidoglycan binding domain (PBD, amino acids 3-39, SEQ ID NO: 3, underlined) and C-terminal catalytic domain of the lysozyme-like superfamily (amino acids 81 - 234, SEQ ID NO: 5, highlighted in gray) are both visualized in the scheme & the sequences.

Figure 2 - SDS-PAGE analysis of the recombinant expression and purification of PVPSE1gp146 (left) and PK-PVPSE1gp147 (right). SDS-PAGE analysis shows the elution protein fractions relative to the LMW reference marker, as well as the flow through (FT) and waste fractions (W). The thick band around 26.4 kDa (for PVPSE1gp146) and 27.7 kDa (for PK-PVPSE1gp146) indicate the high yield of both of the recombinant protein. Some minor secondary bands (protein degradation) are visible together with dimerization of the protein around 52.8 kDa and 55.4 kDa respectively.

Figure 3 - Saturation curves for muralytic activity of *S. enteritis* phage endolysin PVPSE1gp146 in Elution Buffer on outer membrane permeabilized *P. aeruginosa* PAO1. The activity (in $\Delta OD_{655nm}$/min, Y-axis) is shown for increasing concentrations of PVPSE1gp146 (in nM, X-axis). Furthermore, the best linear regression to the linear curve part is indicated as a trend line together with the corresponding optimal R-square values. Results are determined in triplicate. $PAO1_{Krylov}$ substrate is dissolved in an optimal $KH_2PO_4/KH_2PO_4$ buffer.

Figure 4 shows a probable two-dimensional modular structure of modified tag-PVPSE1gp146 variants. The putative N-terminal peptidoglycan binding domain (PBD) and the C-terminal catalytic chitinase domain of the lysozyme-like superfamily of PVP-SE1gp146 are visualized together with the orientation of the N-terminal fused antibacterial

peptide tag (APT).

Figure 5 shows the thermal stability of PVP-SE1gp146 (blue bars) and PK-PVP-SE1gp146 (red bars) on outer membrane permeabilized PAO1 cell substrate after incubation on 42°C (A) and 50°C (B) during different time points in 24h. The muralytic activity is relatively compared to the unheated sample at time 0 and expressed in percentages. Averages and standard deviations of three independent experiments are shown.

Figure 6 shows the thermal stability of PVP-SE1gp146 (A) and PK-PVP-SE1gp146 (B) on outer membrane permeabilized PAO1 cell substrate after incubation between 50 and 100°C during 0 (blue), 20 (red), 40 (green) and 60 (purple) minutes for PVP-SE1gp146; and 0 (blue), 20 (red), 30 (green) and 40 (purple) minutes for PK-PVP-SE1gp146. The muralytic activity is relatively compared to the unheated sample at time 0 and expressed in percentages. Averages and standard deviations of three independent experiments are shown.

**Description**

Definitions:

If appearing herein, the following terms shall have the definitions set out below.

The term "protein" as used herein refers synonymously to the term "polypeptide". The term "protein" as used herein refers to a linear polymer of amino acid residues linked by peptide bonds in a specific sequence. The amino-acid residues of a protein may be modified by e.g. covalent attachments of various groups such as carbohydrates and phosphate. Other substances may be more loosely associated with the polypeptide chains, such as heme or lipid, giving rise to the conjugated proteins which are also comprised by the term "protein" as used herein. The various ways in which the polypeptide chains fold have been elucidated, in particular with regard to the presence of alpha helices and beta-pleated sheets. The term "protein" as used herein refers to all four classes of proteins being all-alpha, all-beta, alpha/beta and alpha plus beta.

The term "fusion protein" as used herein refers to an expression product resulting from the fusion of two nucleic acid sequences. Such a protein may be produced, e.g., in recombinant DNA expression systems. Moreover, the term "fusion protein" as used herein refers to a fusion of a first amino acid sequence as e.g. an endolysin, with a second or further amino acid sequence. The second or further amino acid sequence is preferably a peptide stretch, in particular a cationic peptide or a polycationic peptide. Preferably, said second and/or further amino acid sequence is foreign to and not substantially homologous with any domain of the first amino acid sequence.

The term "peptide stretch" as used herein refers to any kind of peptide linked to a protein such as an endolysin. However, a peptide stretch in the meaning of the present invention does not refer to His6-tags, Strep-tags, Avi-tags, Myc-tags, Gst-tags, JS-tags, cystein-tags, FLAG-tags or other tags known in the art, thioredoxin or maltose binding proteins (MBP). The term "tag" in contrast to the term "peptide stretch" as used herein refers to a peptide which can be useful to facilitate expression and/or affinity purification of a polypeptide, to immobilize a polypeptide to a surface or to serve as a marker or a label moiety for detection of a polypeptide e.g. by antibody binding in different ELISA assay formats as long as the function making the tag useful for one of the above listed facilitation is not caused by the positively charge of said peptide. However, the His6-tag may, depending on the respective pH, also be positively charged, but is used as affinity purification tool as it binds to immobilized divalent cations and is not used as a peptide stretch according to the invention.

The term "peptide" as used herein refers to short polypeptides consisting of from about 2 to about 100 amino acid residues, more preferably from about 4 to about 50 amino acid residues, more preferably from about 5 to about 30 amino acid residues, wherein the amino group of one amino acid residue is linked to the carboxyl group of another amino acid residue by a peptide bond. A peptide may have a specific function. A peptide can be a naturally occurring peptide or a synthetically designed and produced peptide. The peptide can be, for example, derived or removed from a native protein by enzymatic or chemical cleavage, or can be prepared using conventional peptide synthesis techniques (e.g., solid phase synthesis) or molecular biology techniques (see Sambrook, J. et al., Molecular Cloning: A Laboratory Manual, Cold Spring Harbor Press, Cold Spring Harbor, N.Y. (1989)).

The term "endolysin" as used herein refers to an enzyme which is suitable to hydrolyse bacterial cell walls. "Endolysins" comprise at least one "enzymatically active domain" (EAD) having at least one of the following activities: endopeptidase, N-acetyl-muramoyl-L-alanineamidase (amidase), N-acetyl-muramidase, N-acetyl-glucosaminidase (lysozyme) or transglycosylases. In addition, the endolysins may contain also regions which are enzymatically inactive, and bind to the cell wall of the host bacteria, the so-called CBDs (cell wall binding domains). The endolysin may contain two or more CBDs. However, the term "endolysin" as used herein refers also to enzymes having at least one EAD but no CBDs. Generally, the cell wall binding domain is able to bind different components on the surface of bacteria. Preferably, the cell wall binding domain is a peptidoglycan binding domain and binds to the bacteria's peptidoglycan structure. The

different domains of an endolysin can be connected by a domain linker.

The term "domain linker" as used herein refers to an amino acid sequence functioning to connect single protein domains with one another. As a rule domain linkers form no or only few regular secondary structure like $\alpha$-helices or $\beta$-sheets and can occupy different conformations with the respective structural context. Methods to detect domain linker and properties of linker sequences are well known in the art as e.g. described in Bae et al., 2005, Bioinformatics, 21, 2264-2270 or George & Heringa, 2003, Protein Engineering, 15, 871-879.

The term "wild type" or "wt" as used herein refers to the amino acid sequence of the endolysin PVPSE1gp146 as depicted in SEQ ID NO: 1. The nucleic acid sequence encoding the wild type endolysin PVPSE1gp146 is depicted in SEQ ID NO: 2.

The term "deletion" as used herein refers to the removal of 1, 2, 3, 4, 5 or more amino acid or nucleic acid residues from the respective starting sequence.

The term "insertion" or "addition" as used herein refers to the insertion or addition of 1, 2, 3, 4, 5 or more amino acid or nucleid acid residues to the respective starting sequence.

The term "substitution" as used herein refers to the exchange of an amino acid residue located at a certain position for a different one.

The term "cell wall" as used herein refers to all components that form the outer cell enclosure of the Gram-negative bacteria and thus guarantee their integrity. In particular, the term "cell wall" as used herein refers to peptidoglycan, the outer membrane of the Gram-negative bacteria with the lipopolysaccharide, the bacterial cell membrane, but also to additional layers deposited on the peptidoglycan as e.g. capsules, outer protein layers or slimes.

The term "EAD" as used herein refers to the enzymatically active domain of an endolysin. The EAD is responsible for hydrolysing bacterial peptidoglycans. It exhibits at least one enzymatic activity of an endolysin. The EAD can also be composed of more than one enzymatically active module. The term "EAD" is used herein synonymously with the term "catalytic domain".

The present invention relates to a new modular endolysin, isolated from *Salmonella enteritis* phage PVPSE1, useful in the preparation of novel antibacterial agents against Gram-negative bacteria. In particular the present invention relates to a polypeptide comprising an amino acid sequence according to SEQ ID NO: 1 or fragments or derivatives thereof. The polypeptide comprising an amino acid sequence according to SEQ ID NO: 1 is preferably encoded by a nucleotide sequence according to SEQ ID NO: 2.

The endolysin PVPSE1gp146 having an amino acid sequence according to SEQ ID NO: 1 has a length of 236 amino acids. It comprises a N-terminal cell wall binding domain (CBD) and a C-terminal enzymatic active domain (EAD). The N-terminal CBD is a peptidoglycan binding domain (PGB, aa 3 to 39) having an amino acid sequence according to SEQ ID No. 3 and a nucleotide sequence according to SEQ ID No. 4. The C terminal EAD is a catalytic domain (aa 81 to 234) complying with the catalytic domain of the lysozyme-like superfamiliy and having an amino acid sequence according to SEQ ID No. 5 and a nucleotide sequence according to SEQ ID No. 6. The PGB and the catalytic domain of the endolysin PVPSE1gp146 are connected by a domain linker.

Thus, fragments of the polypeptide according to the present invention are polypeptides comprising an amino acid sequence according to SEQ ID NO: 3 and/or according to SEQ ID NO: 5.

The derivatives according to the present invention are polypeptides having a deletion of 1, 2, 3, 4, 5 or more amino acid residues, an addition of 1, 2, 3, 4, 5 or more amino acid residues, an insertion of 1, 2, 3, 4, 5 or more amino acid residues, or a substitution of an amino acid residue located at a certain position for a different one in the amino acid sequence according to SEQ ID NO: 1, 3, and/or 5, and wherein the derivatives exhibit the lytic activity of the PVPSE1gp146 set forth in SEQ ID NO:1.

In one embodiment said modifications and/or alterations of said derivatives of the endolysin according to the present invention can be mutations in particular deletions, insertions, additions, substitutions or any combinations thereof and/or chemical changes of the amino acid residues, e.g. biotinylation, acetylation, pegylation, chemical changes of the amino-, SH-or carboxyl- groups. Said derivatives according to the present invention exhibit the lytic activity of the PVPSE1gp146 (SEQ ID NO: 1) and/or the activity of the fragments according to the present invention. Said activity can be about 10, 20, 30, 40, 50, 60, 70, 80, 90, 100, 110, 120, 130, 140, 150, 160, 170, 180, 190 or about 200 % of the activity of the PVPSE1gp146 and/or the activity of the fragments according to the present invention. The activity can be measured by assays well known in the art by a person skilled in the art as e.g. the plate lysis assay or the liquid lysis assay which are e.g. described in (Briers et al., J. Biochem. Biophys Methods 70: 531-533, (2007)).

In a preferred embodiment of the present invention the polypeptide, fragment and/or derivative according to the present invention comprises additionally a tag such as a His6-tag, Strep-tag, Avi-tag, Myc-tag, Gst-tag, JS-tag, cystein-tag, FLAG-tag or other tags known in the art at the N-terminus or at the C-terminus. In a preferred embodiment of the present invention said tag is linked to the polypeptide, fragment and/or derivative according to the present invention at the C-terminus. Said tag may be linked to said polypeptide, fragment and/or derivative over additional amino acid residues. Said additional amino acid residues may be consist of at least 1, 2, 3, 4, 5, 6, 7, 8, 9 or 10 additional amino acid residues. In a preferred embodiment of the present invention the tag is linked to the polypeptide, fragment and/or

derivative according to the present invention by the additional amino acid residues Leu-Glu or Lys-Gly. In a preferred embodiment the present invention relates to a polypeptide comprising an amino acid sequence according to SEQ ID No. 7. The polypeptide having an amino acid sequence according to SEQ ID No. 7 comprises in comparison to the polypeptide having an amino acid sequence according to SEQ ID No.1, respectively, an additional C-terminal His6-tag linked to the C terminus of the polypeptide having an amino acid sequence according to SEQ ID No.1, by the additional amino acid residues lysine and glycine (Lys-Gly). In addition, a *Bam*HI restriction site was introduced at position 2 and 3. A polypeptide comprising an amino acid sequence according to SEQ ID No. 7 is preferably encoded by a nucleotide sequence according to SEQ ID No. 8.

A further aspect of the present invention are fusion proteins composed of a polypeptide, fragment and/or derivative according to the present invention and a peptide stretch with membrane disrupting or LPS disrupting activity fused to the polypeptide, fragment and/or derivative according to the present invention at the N- or C-terminus to enhance the activity of said endolysin against Gram negative bacteria. Preferably, said peptide stretch is a cationic peptide and/or a polycationic peptide according to WO2010023207.

The peptide stretch of the fusion protein according to the present invention is preferably covalently bound to the polypeptide, fragment and/or derivative according to the present invention. Preferably, said peptide stretch consists of at least 5, more preferably at least of 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 or at least 100 amino acid residues. Especially preferred is a peptide stretch comprising 5 to 100 amino acid residues, 5 to 50 or 5 to 30 amino acid residues. More preferred is a peptide stretch comprising about 6 to about 42 amino acid residues, about 6 to about 39 amino acid residues, about 6 to about 38 amino acid residues, about 6 to about 31 amino acid residues, about 6 to about 25 amino acid residues, about 6 to about 24 amino acid residues, about 6 to about 22 amino acid residues, about 6 to about 21 amino acid residues, about 6 to about 20 amino acid residues, about 6 to about 19 amino acid residues, about 6 to about 16 amino acid residues, about 6 to about 14 amino acid residues, about 6 to about 12 amino acid residues, about 6 to about 10 amino acid residues or about 6 to about 9 amino acid residues. Preferably, the peptide stretch is no tag such as a His6-tag, Strep-tag, Avi-tag, Myc-tag, Gsttag, JS-tag, cystein-tag, FLAG-tag or other tags known in the art and no thioredoxin or maltose binding proteins (MBP). However, the peptide stretch may comprise in addition such tag or tags or the like, which are used to purify or locate proteins.

The present invention further relates to an isolated nucleic acid molecule encoding the polypeptide, fragment, derivative and/or fusion protein according to the present invention. Especially preferred isolated nucleic acid molecules according to the present invention comprise a nucleic acid sequence according to SEQ ID NO: 2, 4, 6 or 8. The present invention further relates to a vector comprising the nucleic acid molecule according to the present invention. Said vector may provide for the constitutive or inducible expression of said polypeptide, fragment, derivative and/or fusion protein according to the present invention. The invention also relates to a method for obtaining said polypeptide, fragment, derivative and/or fusion proteins from a micro-organism, such as a genetically modified suitable host cell which expresses said polypeptide, fragment, derivative and/or fusion proteins. Said host cell may be a micro-organism such as bacteria or yeast or an animal cell as e.g. a mammalian cell, in particular a human cell. In one embodiment of the present invention the host cell is an *Escherichia coli* cell. The host may be selected due to mere biotechnological reasons, e.g. yield, solubility, costs, etc. but may be also selected from a medical point of view, e.g. a nonpathological bacteria or yeast or human cells. Another aspect of the present invention is related to a method for genetically transforming a suitable host cell in order to obtain the expression of the polypeptide, fragment, derivative and/or fusion proteins according to the present invention, wherein the host cell is genetically modified by the introduction of a genetic material encoding said polypeptide, fragment, derivative and/or fusion proteins into the host cell and obtain their translation and expression by genetic engineering methods well known by the man skilled in the art.

In a further aspect the present invention relates to a composition, preferably a pharmaceutical composition, comprising a polypeptide, fragment, derivative and/or fusion protein according to the present invention and/or a host transformed with a nucleic acid molecule or a vector comprising a nucleotide sequence encoding a polypeptide, fragment, derivative and/or fusion protein according to the present invention.

In a preferred embodiment of the present invention the composition comprises additionally agents permeabilizing the outer membrane of Gram-negative bacteria such metal chelators as e.g. EDTA, TRIS, lactic acid, lactoferrin, polymyxin, citric acid and/or other substances as described e.g. by Vaara (Agents that increase the permeability of the outer membrane. Vaara M. Microbiol Rev. 1992 Sep;56(3):395-441). Also preferred are compositions comprising combinations of the above mentioned permeabilizing agents. Especially preferred is a composition comprising about 10 μM to about 100 mM EDTA, more preferably about 50 μM to about 10 mM EDTA, more preferably about 0.5 mM to about 10 mM EDTA, more preferably about 0.5 mM to about 2 mM EDTA, more preferably about 0.5 mM to about 1 mM EDTA. Also preferred is a composition comprising about 0.5 mM to about 2 mM EDTA, more preferably about 1 mM EDTA and additionally about 10 to about 100 mM TRIS.

The present invention also relates to a polypeptide, fragment, derivative and/or fusion protein according to the present invention and/or a host transformed with a nucleic acid comprising a nucleotide sequence encoding a polypeptide, fragment, derivative and/or fusion protein according to the present invention for use as a medicament. In a further aspect the present invention relates to the use of a polypeptide, fragment, derivative and/or fusion protein according to the present invention and/or a host transformed with a vector comprising a nucleic acid molecule comprising a nucleotide sequence encoding a polypeptide, fragment, derivative and/or fusion protein according to the present invention in the manufacture of a medicament for the treatment and/or prevention of a disorder, disease or condition associated with Gram-negative bacteria. In particular the treatment and/or prevention of the disorder, disease or condition may be caused by Gram-negative bacteria of bacterial groups, families, genera or species comprising strains pathogenic for humans or animals like *Enterobacteriaceae* (*Escherichia,* especially *E. coli, Salmonella, Shigella, Citrobacter, Edwardsiella, Enterobacter*, *Hafnia, Klebsiella, especially K. pneumonia, Morganella*, *Proteus, Providencia*, *Serratia, Yersinia*), *Pseudomonadaceae* (*Pseudomonas, especially P. aeruginosa*, *Burkholderia, Stenotrophomonas, Shewartella, Sphingomonas*, *Comamonas*), *Neisseria, Moraxella, Vibrio, Aeromonas*, *Brucella, Francisella*, *Bordetella, Legionella*, *Bartonella*, *Coxiella, Haemophilus, Pasteurella, Mannheimia*, *Actinobacillus*, *Gardnerella*, *Spirochaetaceae (Treponema and Borrelia), Leptospiraceae, Campylobacter, Helicobacter, Spirillum, Streptobacillus, Bacteroidaceae (Bacteroides, Fusobacterium, Prevotella, Porphyromonas*), *Acinetobacter*, especially *A. baumanii.* In particular, the treatment and/or prevention of the disorder, disease or condition may be caused by *Pseudomonas aeruginosa, Pseudomonas putida, Burkholderia pseudomallei, E. coli* and/or *Salmonella typhimurium*.

The present invention further relates to a medicament comprising a polypeptide, fragment, derivative and/or fusion protein according to the present invention and/or a host transformed with a nucleic acid comprising a nucleotide sequence encoding a polypeptide, fragment, derivative and/or fusion protein according to the present invention.

In a further aspect the present invention relates to an inventive polypeptide or fusion protein for use in a method of treating a disorder, disease or condition in a subject in need of treatment and/or prevention, which method comprises administering to said subject an effective amount of a polypeptide, fragment, derivative and/or fusion protein according to the present invention and/or an effective amount of a host transformed with a nucleic acid comprising a nucleotide sequence encoding a polypeptide, fragment, derivative and/or fusion protein according to the present invention or a composition according to the present invention. The subject may be a human or an animal. In particular said method of treatment may be for the treatment and/or prevention of infections of the skin, of soft tissues, the respiratory system, the lung, the digestive tract, the eye, the ear, the teeth, the nasopharynx, the mouth, the bones, the vagina, of wounds of bacteraemia and/or endocarditis caused by Gram-negative bacteria, in particular by the Gram-negative bacteria as listed above.

The dosage and route of administration used in a method of treatment (or prophylaxis) depends on the specific disease/site of infection to be treated. The route of administration may be for example oral, topical, nasopharyngeal, parenteral, intravenous, rectal or any other route of administration. For application of a polypeptide, fragment, derivative and/or fusion protein according to the present invention and/or an effective amount of a host transformed with a nucleic acid comprising a nucleotide sequence encoding a polypeptide, fragment, derivative and/or fusion protein according to the present invention or a composition according to the present invention to a site of infection (or site endangered to be infected) a formulation may be used that protects the active compounds from environmental influences such as proteases, oxidation, immune response etc. until it reaches the site of infection. Therefore, the formulation may be capsule, dragee, pill, suppository, injectable solution or any other medical reasonable galenic formulation. Preferably, the galenic formulation may comprise suitable carriers, stabilizers, flavourings, buffers or other suitable reagents. For example, for topical application the formulation may be a lotion or plaster, for nasopharyngeal application the formulation may be saline solution to be applied via a spray to the nose.

Preferably, a polypeptide, fragment, derivative and/or fusion protein according to the present invention is used for medical treatment, if the infection to be treated (or prevented) is caused by multiresistant bacterial strains, in particular by strains resistant against one or more of the following antibiotics: streptomycin, tetracycline, cephalothin, gentamicin, cefotaxime, cephalosporin, ceftazidime or imipenem. Furthermore, a polypeptide, fragment, derivative and/or fusion protein according to the present invention can be used in methods of treatment by administering it in combination with conventional antibacterial agents, such as antibiotics, lantibiotics, bacteriocins or endolysins, etc.

The present invention also relates to a pharmaceutical pack comprising one or more compartments, wherein at least one compartment comprises one or more polypeptide, fragment, derivative and/or fusion protein according to the present invention and/or one or more hosts transformed with a nucleic acid comprising a nucleotide sequence encoding a polypeptide, fragment, derivative and/or fusion protein according to the present invention or a composition according to the present invention.

In another aspect the present invention relates to a process of preparation of a pharmaceutical composition, said process comprising admixing one or more polypeptide, fragment, derivative and/or fusion protein according to the present invention and/or one or more hosts transformed with a nucleic acid comprising a nucleotide sequence encoding a polypeptide, fragment, derivative and/or fusion protein according to the present invention with a pharmaceutically

acceptable diluent, excipient or carrier.

In an even further aspect the composition according to the present invention is a cosmetic composition. Several bacterial species can cause irritations on environmentally exposed surfaces of the patient's body such as the skin. In order to prevent such irritations or in order to eliminate minor manifestations of said bacterial pathogens, special cosmetic preparations may be employed, which comprise sufficient amounts of the polypeptide, fragment, derivative and/or fusion protein according to the present invention in order to degrade already existing or freshly settling pathogenic Gram-negative bacteria.

In a further aspect the present invention relates to the polypeptide, fragment, derivative and/or fusion protein according to the present invention for use as diagnostic means in medicinal, food or feed or environmental diagnostics, in particular as a diagnostic means for the diagnostic of bacteria infection caused in particular by Gram-negative bacteria. In this respect the polypeptide, fragment, derivative and/or fusion protein according to the present invention may be used as a tool to specifically degrade pathogenic bacteria, in particular Gram-negative pathogenic bacteria. The degradation of the bacterial cells by the polypeptide, fragment, derivative and/or fusion protein according to the present invention can be supported by the addition of detergents like Triton X-100 or other additives which weaken the bacterial cell envelope like polymyxin B. Specific cell degradation is needed as an initial step for subsequent specific detection of bacteria using nucleic acid based methods like PCR, nucleic acid hybridization or NASBA (Nucleic Acid Sequence Based Amplification), immunological methods like IMS, immunofluorescence or ELISA techniques, or other methods relying on the cellular content of the bacterial cells like enzymatic assays using proteins specific for distinct bacterial groups or species (e.g. β-galactosidase for enterobacteria, coagulase for coagulase positive strains). In a further aspect the present invention relates to the use of the polypeptide, fragment, derivative and/or fusion protein according to the present invention for the treatment or prevention of Gram-negative bacterial contamination of foodstuff, of food processing equipment, of food processing plants, of surfaces coming into contact with foodstuff such as shelves and food deposit areas and in all other situations, where pathogenic, facultative pathogenic or other undesirable bacteria can potentially infest food material, of medical devices and of all kind of surfaces in hospitals and surgeries.

In particular, a polypeptide, fragment, derivative and/or fusion protein of the present invention may be used prophylactically as sanitizing agent. Said sanitizing agent may be used before or after surgery, or for example during hemodialysis. Moreover, premature infants and immunocompromised persons, or those subjects with need for prosthetic devices may be treated with a fusion protein according to the present invention. Said treatment may be either prophylactically or during acute infection. In the same context, nosocomial infections, especially by antibiotic resistant strains like *Pseudomonas aeruginosa* (FQRP), *Acinetobacter* species and *Enterobacteriaceae* such as *E.coli, Salmonella, Shigella*, *Citrobacter, Edwardsiella*, *Enterobacter, Hafnia, Klebsiella*, *Morganella*, *Proteus*, *Providencia*, *Serratia* and *Yersinia* species may be treated prophylactically or during acute phase with a polypeptide, fragment, derivative and/or fusion protein of the present invention. Therefore, a polypeptide, fragment, derivative and/or fusion protein according to the present invention may be used as a disinfectant also in combination with other ingredients useful in a disinfecting solution like detergents, tensids, solvents, antibiotics, lantibiotics, or bacteriocins.

The following examples explain the present invention but are not considered to be limiting to the disclosure or the appended claims. Unless indicated differently, molecular biological standard methods were used, as e.g., described by Sambrock et al., 1989, Molecular Cloning: A Laboratory Manual, 2nd edition, Cold Spring Harbor Laboratory Press, Cold Spring Harbor, New York.

**<u>Example 1</u>: PVP-SE1gp146 and its N-terminal PK-fused variant PK-PVP-SE1gp146**

**Cloning, expression and purification of the modular endolysin PVP-SE1gp146 of the *Salmonella enteritidis* phage PVPSE1 and its N-terminal PK-fused variant PK-PVP-SE1gp146**

PVP-SE1gp146 (236 amino acids long, MW = 25325 Da) is the modular endolysin originating from *Salmonella enteritidis* phage PVPSE1, predicted to possess an N-terminal peptidoglycan binding domain (from amino acids 3 to 39) and a C-terminal catalytic chitinase domain of the lysozyme-like superfamily (from amino acids 81 to 234) (see Figure 1).

Purified genomic DNA of phage PVPSE1 (obtained from Dr. S. Santos, Universidade do Minho, Braga, Portugal) was used as a template for the amplification of the open reading frame (ORF146) encoding the endolysin PVP-SE1gp146 in a standard PCR reaction with *Pfu* polymerase (Fermentas, Ontario, Canada). The following PCR parameters were used:

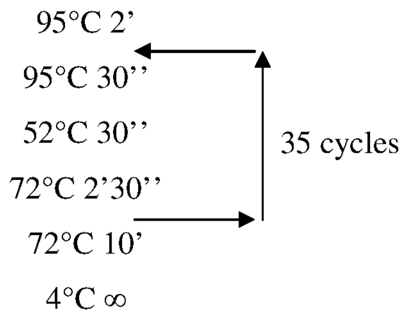


Forward (Primer 1) and reverse (Primer 2) primers for this PCR are shown in **Table 1**.

**Table 1 - Primers used during standard PCR amplification of ORF146 and during tail PCR for addition of an N-terminal PK tag to ORF146**

| # Primer name | Primer sequence |
|---|---|
| 1. ORF146 forward primer | ATGGGATCCAATGCTGCAATTGCGGAGA<br>**SEQ ID NO:9** |
| 2. ORF146 reverse primer | CGAGGTTAGAACAGATTTTGCCT<br>**SEQ ID NO:10** |
| 3.extended ORF146forward primer | ATGGGATCC**AAACGCAAGAAACGTAAGAAACGCAAAA**ATGCTGCAATTGCGGAGA<br>**SEQ ID NO:11** |

To extend the 5' end of ORF146 with a gene fragment encoding the polycationic 9-mer peptide *Lys-Arg-Lys-Lys-Arg-Lys-Lys-Arg-Lys-* a tail PCR (following the same parameters as for the standard PCR above) with an extended 5' primer (Primer 3, SEQ ID NO:11, **Table 1**) and the standard 3' primer (Primer 2, SEQ ID NO:10, **Table 1**). Both obtained PCR fragments were then ligated in the commercial available pEXP5CT/TOPO® expression vector (Invitrogen, Carlsbad, CA, USA) following the TA-cloning protocol provided by the manufacturer, causing a fusion of the 3'/C-terminal side of the endolysin ORF/protein to the 6xHistidine tag necessary for purification. The obtained DNA and amino acid sequences for the recombinant PVP-SE1gp146 and PK-PVP-SE1gp146 endolysins are shown in SEQ ID NO:7 (amino acid sequence of recombinant PVPSE1gp146), SEQ ID NO:8 (nucleotide sequence of recombinant PVPSE1gp146), SEQ ID NO:12 (DNA sequence of recombinant PK-PVPSE1gp146) and SEQ ID NO:13 (amino acid sequence of recombinant PK-PVPSE1gp146).

Recombinant expression of PVP-SE1gp146/PK-PVP-SE1gp146 was performed in exponentially growing *E. coli* BL21 (λDE3) pLysS cells after induction with 1 mM IPTG (isopropylthiogalactoside) at 16°C overnight. The endolysin was then purified by $Ni^{2+}$ affinity chromatography (Akta FPLC, GE Healthcare) using the C-terminal 6xHis-tag, encoded by the pEXP5CT/TOPO® expression vector. The $Ni^{2+}$ affinity chromatography is performed in 4 subsequent steps, all at room temperature:

1. Equilibration of the Histrap HP 1 ml column (GE Healthcare) with 10 column volumes of Washing Buffer (70 mM imidazole, 0.5 mM NaCl and 20 mM $NaH_2P0_4$-NaOH on pH 7.4) at a flow rate of 0.5 ml/min.
2. Loading of the total lysate (with the desired endolysin) on the Histrap HP 1 ml column at a flow rate of 0.5 ml/min.
3. Washing of the column with 15 column volumes of Washing Buffer at a flow rate of 1 ml/min.
4. Elution of bounded endolysin from the column with 10 column volumes of Elution Buffer (500 mM imidazole, 5 mM NaCl and 20 mM $NaH_2P0_4$-NaOH on pH 7.4) at a flow rate of 0.5 ml/min

The yields for the purifications of recombinant PVP-SE1gp146/PK-PVP-SE1gp146 are shown in **Table 2**. The protein concentration was determined spectrophotometrically at a wavelength of 280 nm. Purified stock solutions of both proteins in Elution Buffer (20 mM $NaH_2P0_4$-NaOH pH7.4; 0.5 M NaCl; 500 mM imidazole) were over 90% pure as determined visually on an SDS-PAGE gel (**Figure 2**).

**Table 2 - Yields of purified recombinant PVP-SE1gp146/PK-PVP-SE1gp146 endolysins per liter *E. coli* expression culture as determined by spectrophotometric measurement at 280 nm.** The yield of the PK modified variant is almost twice as high as the unmodified PVP-SE1gp146, revealing a stabilizing effect of the PK tag on protein expression.

| Endolysin | Expression yield |
|---|---|
| PVP-SE1gp146<br>PK-PVP-SE1gp146 | 38.18 mg/L<br>65.15 mg/L |

**Example 2: Characterization and determination of biochemical muralytic activity of Salmonella enteritis phage endolysin PVPSE1gp146**

For quantification of muralytic activity, outer membrane permeabilized *P. aeruginosa* $PAO1_{krylov}$ ($PAO1_{Krylov}$ obtained from prof V. Krylov from the State Institute for Genetics of Industrial Microorganisms, 1st Dorozhnii proezd 1, 113545 Moscow, Russia) cell substrate, sensitized for endolysin activity, was used. In presence of an access of this substrate, a saturation curve for enzymatic activity of PVPSE1gp146 in Elution Buffer was created showing the peptidoglycan degrading activity (expressed as the drop of $OD_{655nm}$ per minute) in function of a range of different endolysin concentrations (expressed in nM) (Figure 3). Measurements were done in triplicate with the substrate dissolved in an optimal $KH_2PO_4/K_2HPO_4$ buffer for enzymatic activity with a pH of 7.3 and an ionic strength of 80 mM.

The slope of the best linear regression to the linear part of this saturation curve is used for determination of the muralytic activity value according to an adapted versions of the definition for enzymatic activity by Cheng *et al.* (2004) and Briers *et al.* (2007):

$$\text{Activity (in units/mM)} = \frac{(\text{Slope } (\Delta OD_{655nm}/\text{min}))\ /\ \text{mM}}{0.001}$$

Using the upper formula, PVPSE1gp146 possesses a muralytic activity value of 15,005,330 units/mM when dissolved in Elution Buffer.

**Example 3: N-terminal antibacterial peptide fusion to endolysin of *Salmonella enteritidis* phage PVP-SE1**

**1. Cloning, expression and purification of antibacterial tag fused PVP-SE1gp146 constructs**

PVP-SE1gp146 was N-terminally fused to a set of natural antibacterial peptide tags (shown in **Table 3**) in order to higher its anti-Gram-negative activity and to broaden its bacterial host range. These tags have been selected or developed based on their amphipathic, hydrophobic or polycationic properties and short length. This list contains a number of known antibacterial peptides selected in literature which are derived from insects, amphibians or fish and prove to work efficiently on Gram-negative strains; and three designed antibacterial tags. **Figure 4** illustrates a probable two-dimensional modular structure of modified tag-PVPSE1gp146 variants.

**Table 3 - List of antibacterial peptide tags fused to PVP-SE1gp146**

| Tag | Description + size | Amino acid sequence | Reference |
|---|---|---|---|
| α4-helix of T4-lysozyme | Amphipathic helix (13 aa) | PNRAKRVITTFRT (SEQ ID NO: 14) | Matthews* |
| Pentapeptide (designed) | Hydrophobic (5 aa) | FFVAP (SEQ ID NO:15) | Briers *et al.*, 2008 |
| Artilysin 1 (designed) | Hydrophobic (18 aa) | GFFIPAVILPSIAFLIVP (SEQ ID NO: 16) | Walmagh |
| Artilysin 2 (designed) | Amphipathic helix (25 aa) | GKPGWLIKKALVFKKLIRRPLK RLA (SEQ ID NO:17) | Walmagh |

(continued)

| Tag | Description + size | Amino acid sequence | Reference |
|---|---|---|---|
| Parasin 1 | Alpha-helical peptide (19 aa) | KGRGKQGGKVRAKAKTRSS (SEQ ID NO:18) | Park, Y *et al.*, 1998 |
| Lycotoxin 1 | Amphiphatic helix (25 aa) | IWLTALKFLGKHAAKKLAKQQ LSKL (SEQ ID NO:19) | Yan & Adams, 1988 |

Except for the pentapeptide (PP) tag, all antibacterial peptide tags were fused to the ORF which encodes for PVP-SE1gp146 using an adapted version of the Ligation Independent Cloning (LIC) technique (Berrow et al., 2007, A versatile ligation-independent cloning method suitable for high-throughput expression screening applications). Therefore, an unique Ec1136II restriction site was inserted in front of the PVP-SE1gp146 encoding gene by a tail PCR with a specific designed 5' forward primer (GGAATGGGGAGCTCCTCCAATGCTGCAATTGCGGAGAT; SEQ ID NO:30) and the standard PVP-SE1gp146 reverse primer (CGAGGTTAGAACAGATTTTGCCT, SEQ ID NO: 10) on pure genomic DNA of phage OBP. This extended fragment was then ligated in the pEXP5CT/TOPO® expression vector (Invitrogen, Carlsbad, CA, USA) by following the TA cloning protocol of the manufacturer. Pure plasmid was cutted once in an Ecl136II restriction digest and hybridized peptide cassettes (created by hybridization of primer pairs, see **Table 4**) were inserted into the cutted plasmid without a necessary ligation step. For the N-terminal pentapeptide tag fusion a tail PCR with an extended 5' primer which encodes for this pentapeptide (ATGGGATCCTTCTTCGTAGCACCGGGCTCCTCCAATGCTGCAAT, SEQ ID NO:31; pentapeptide underlined) was applied on purified pEXP5-CT/PK-OBPgpLys plasmid DNA. Correct insertion of the fragments in the expression vector was verified by sequencing analysis before introducing the construct into a suitable *Escherichia coli* BL21(DE3)pLysS expression strain.

**Table 4 - Primers used for hybridization of antibacterial peptide tags before fusion to PVP-SE1gp146**

| Tag | Forward primer | Reverse primer |
|---|---|---|
| α4-helix of T4-lysozyme | TTGGAATGGGGAGCCCGAACCGTGCAAAACGTGTAATCA (SEQ ID NO:20) | CATTGGAGGAGCCGGTACGGAAGGTGGTGATTACACGTT (SEQ ID NO:21) |
| Artilysin 1 (designed) | TTATGGGCTTCTTCATCCCGGCAGTAATCCTGCCCTCCA (SEQ ID NO:22) | CATTGGAGGAGCCCGGTACGATCAGGAATGCGATGGAGGGCAGGAT (SEQ ID NO:23) |
| Artilysin 2 (designed) | TTATGGGCAAACCGGGCTGGCTGATCAAAAGGCACTGGTATTCAAGA (SEQ ID NO:24) | CATTGGAGGAGCCTGCCAGTCTCTTCAGCGGACGACGGATCAGTTTCTTGAATACCA (SEQ ID NO:25) |
| Parasin 1 | TTGGAATGGGGAGCAAAGGCCGTGGCAAGCAGGGAGGCAAAGTACGTG (SEQ ID NO:26) | CATTGGAGGAGCCTGAGGAACGGGTCTTTGCTTTTGCACGTACTTTGC (SEQ ID NO:27) |
| Lycotoxin 1 | GGAATGGGGAGCATCTGGCTGACCGCACTGAAATTCCTCGGCAAACACGCCGCAA (SEQ ID NO:28) | CATTGGAGGAGCCCAGTTTGGATAATTGCTGTTTTGCCAGTTTCTTTGCGGCGTGTT (SEQ ID NO:29) |

Standard expression is performed in Lysogeny Broth (LB) in exponentially growing cells ($OD_{600nm}$ = 0.6) induced with 1 mM isopropyl-beta-D-thiogalactopyranoside. Expression parameters like temperature, time and expression strain varied on a protein specific basis in order to optimize the soluble expression levels of the modified endolysins (see Table 5). For purification, cells from an expression culture (500-600 ml) are harvested (4500 rpm, 30 min, 4°C) and resuspended in 1/25 volumes of lysis buffer (10 mM imidazole, 20 mM $NaH_2PO_4$, 0.5 M NaCl, pH 7.4). This suspension is frozen/thawed three times prior to sonication (8 x 30 s, amplitude 40 % on a Vibra CellTM, Sonics, Dandurry, CT, USA) and filtered through 0.45 and 0.22 μm Durapore membrane filters (Millipore, Billerica, MA, USA). Purification of the His-tagged fusion protein was performed by a one-step protocol employing $Ni^{2+}$-affinity chromatography (HisTrap HP 1 ml column, GE Healthcare, Buckinghamshire, UK) according to the manufacturer's instructions. The $Ni^{2+}$ affinity chromatography is performed in 4 subsequent steps, all on room temperature:

1. Equilibration of the Histrap HP 1 ml column (GE Healthcare) with 10 column volumes of Washing Buffer (protein dependent amount of imidazole (60-70 mM), 0.5 mM NaCl and 20 mM $NaH_2P0_4$-NaOH on pH 7.4) at a flow rate of 0.5 ml/min.
2. Loading of the total lysate (with the wanted endolysin) on the Histrap HP 1 ml column at a flow rate of 0.5 ml/min.
3. Washing of the column with 15 column volumes of Washing Buffer at a flow rate of 1 ml/min.
4. Elution of bounded endolysin from the column with 10 column volumes of Elution Buffer (500 mM imidazole, 5 mM NaCl and 20 mM $NaH_2P0_4$-NaOH on pH 7.4) at a flow rate of 0.5 ml/min

The wash buffer included a low imidazole concentration which varied on protein specific base to ensure higher purity of the protein (see **Table 5**). The total yields of recombinant proteins per liter E. coli expression culture is also shown in **Table 5**. The values were determined by spectrophotometric measurement of the protein concentration and the total volume of the purified stock solution at a wavelength of 280 nm. Purified stock solutions were at least 90% pure as determined visually on SDS-PAGE gels (data not shown).

**Table 5 - Expression parameters and obtained protein yields per liter expression culture of N-terminal fused PVP-SE1gp146 constructs.** ON = overnight;* = *E. coli* Codon Plus RIL, Agilent Technologies Catalog nr.230245, genotype = *E. coli* B F- *ompT* hsdS($r_B^-$ $m_B^-$) *dcm*+ $Tet^r$ gal λ(DE3) *endA* Hte [*argU ileY leuW* $Cam^r$]

| Modified endolysin | Temperature/ time | [imidazole] (in mM) | Strain | Protein Yield (in mg/l) |
|---|---|---|---|---|
| **α4-PVP-SE1gp146** | 16°C/ON | 60 | *E. coli* Codon Plus RIL* | 1.5 |
| **PP-PVP-SE1gp146** | 16°C/ON | 70 | *E. coli* Codon Plus RIL* | 4.3 |
| **Artilysin1-PVP-SE1gp146** | 16°C/ON | 65 | *E. coli* Codon Plus RIL* | 5.1 |
| **Artilysin2- PVP-SE1gp146** | 16°C/ON | 70 | *E. coli* Codon Plus RIL* | 3.2 |
| **Parasin1- PVP-SE1gp146** | 16°C/ON | 60 | *E. coli* Codon Plus RIL* | 2.4 |
| **Lycotoxin1-PVP-SE1gp146** | 16°C/ON | 60 | *E. coli* Codon Plus RIL* | 1.7 |

**2. *In vitro* antibacterial activity of N-terminal tag fused PVP-SE1gp146 variants**

To determine if the antibacterial tag fusions have a positive effect on the *in vitro* antibacterial activity of PVP-SE1gp146, some of the N terminal tag fused PVP-SE1gp146 variants were tested on 2 different Gram-negative bacteria: *Escherichia coli* XL1 blue and the food pathogen *Salmonella typhimurium* LT2 for Artilys2-PVPSE1gp146 and Lycotox1-PVPSE1gp146. To optimize their antibacterial properties small amounts of the outer membrane permeabilizer di sodium ethylene diamino tetra acetic acid ($EDTA-Na_2$) were added.

Exponential growing Gram-negative bacterial cells ($OD_{600nm}$ = 0.6) were 100-fold diluted to a final density of about $10^6$ cells/ml and incubated for 30 minutes at room temperature without shaking with 5 μM of the different modified PVP-SE1gp146 variants with and without 0.5 mM of $EDTA-Na_2$. After incubation cell suspensions were diluted three times starting with $10^6$ cells/ml to $10^5$ cells/ml then to a concentration of $10^4$ cells/ml and finally to a concentration of $10^3$

cells/ml and 100 μl of each dilution was plated out on LB-medium.

The residual colonies were counted after an overnight incubation on 37°C. Based on the counted cell numbers the antibacterial activity as the relative inactivation in logarithmic units ($log_{10}(N_0/N_i)$ with No = number of untreated cells and $N_i$ = number of treated cells, both counted after incubation) is calculated (**Table 6**).

**Table 6 - *In vitro* antibacterial activity of 5 μM of the N-terminal tag fused PVP-SE1gp146 variants without (A) and with (B) 0.5 mM EDTA-$Na_2$ on Gram-negative bacteria compared to the unmodified PVP-SE1gp146.** The antibacterial activity was quantified as the relative inactivation in logarithmic units (= $log_{10}(N_0/N_i)$ with No = number of untreated cells and $N_i$ = number of treated cells counted after incubation). All samples were replicated in threefold. Averages +/- standard deviations are shown. The maximal reduction observed is dependent on the detection level of 10 cells/ml and the initial cell density.

| | | | | | |
|---|---|---|---|---|---|
| A) | | | | | |
| | | *S. typhimurium* | | *E. coli* | |
| Artilys2-PVPSE1gp146 | | 0.12 | 0.12 | 0.10 | 0.09 |
| PVPSE1gp146 | | 0.19 | 0.10 | 0.10 | 0.09 |
| | | | | | |
| B) | | | | | |
| | | *S. typhimurium* | | *E. coli* | |
| Artilys2-PVPSE1gp146 | EDTA | 0.41 | 0.25 | 0.16 | 0.10 |
| Lycotox1-PVPSE1gp146 | EDTA | 0.87 | 0.28 | 0.34 | 0.15 |
| PVPSE1gp146 | EDTA | 0.44 | 0.11 | 0.11 | 0.06 |

Without the outer membrane permeablilizer EDTA, only a low antibacterial activity is detectable in *S. typhimurium* and *E. coli.* In samples including EDTA a stronger antibacterial activity can be observed. The fusion protein with the modification Artilysin 2 possess good antibacterial activity against *S. typhimurium.* However, the activity is in the range of the unmodified variant PVPSE1gp146. The antibacterial activity against *S. typhimurium* is even improved with the fusion protein with the Lycotoxin 1 modification. The Lycotoxin fusion protein as well as the Artilysin 2 fusion protein exert also antibacterial activity in view of *E. coli,* whereby the activity is on a reduced level compared to the antibacterial activity against *S. typhimurium.*

<u>**Example 4:**</u> **Thermal stability of recombinant PVP-SE1gp146 and its PK fused variant PK-PVP-SE1gp146**

Temperature plays a major role in stability and thus the enzymatic or muralytic activity of PVP-SE1gp146/PK-PVP-SE1gp146. Raising temperatures can easily break hydrogen bonds and non-polar hydrophobic interactions inside the protein's three dimensional structure, causing denaturation of the protein and partly loss of its enzymatic function. To investigate the thermal stability of PVP-SE1gp146 and PK-PVP-SE1gp146, both recombinant endolysins are incubated on different temperatures (42, 50, 60, 70, 80, 90 and 100°C) for different time-intervals (1, 2, 3, 4, 8 and 24 hours on 42 and 50°C; each 10 minutes during 1 hour for 50 to 100°C) in a Biometra T3000 Thermocycler (Göttingen, Germany). After thermal incubation 30 μl of each incubated sample is added to 270 μl of outer membrane permeabilized *P. aeruginosa* PAO1 ($PAO1_{Krylov}$ obtained from prof V. Krylov from the State Institute for Genetics of Industrial Microorganisms, 1st Dorozhnii proezd 1, 113545 Moscow, Russia) cell substrate (Lavigne *et al.,* 2004**), and the optical density ($OD_{655nm}$) is followed in function of time. Each sample is incubated and tested in triplicate. Enzymatic or muralytic activity of each sample is quantified based on the $OD_{655nm}$/time curve according to the Briers *et al.* (2007)*** paper and expressed in percentages relative to the activity of unheated reference sample at time 0 (= 100 % activity). In **Figure 5**, the results for incubation on 42°C and 50°C during time-interval of 24 hours are shown. The value of 42°C is selected because it mimics the fever body temperature condition, whereas 50°C is the critical temperature by which thermolabile proteins become denaturized.

Both PVP-SE1gp146 and PK-PVP-SE1gp146 are thermostable on 42°C (**Figure 5A**) even after 24h of incubation. Even at 50°C (**Figure 5B**), the muralytic activity of PVP-SE1gp146 does not change significantly over a time period of 24h. Contrarily, PK-PVP-SEg1gp146 loses 30% of the initial activity between time points of 8 and 24 h, probably due to the presence of the PK tag. After 2h of incubation at 42°C and 3h at 50°C the muralytic activity of PK-PVP-SE1gp146 even raises significantly. Possibly, the higher temperature initially inhibits the reduction in activity caused by

the PK modification; an effect that diminishes with increasing incubation time.

Because PVP-SE1gp146 still shows complete activity on 50°C after 24 h, the thermal stability of PVP-SE1gp146/PK-PVP-SE1gp146 was also determined at higher temperatures between 50 and 100°C during an incubation time of 1 h and 40 minutes respectively (**Figure 6**). Activity is measured for PVP-SE1gp146 at time points 0, 20, 40 and 60 minutes (**Figure 6A**) and for PK-PVP-SE1gp146 at 0, 20, 30 and 40 minutes (**Figure 6B**).

**Figure 6A** shows that PVP-SE1gp146 still keeps its maximal activity after incubation at 80°C for 1h, but at 100°C a short incubation of 20 minutes is enough to reduce its activity with more than 80%. As described in **Figure 5** above, the activity of PK-PVP-SE1gp146 gradually reduces at temperatures higher than 50°C. After 30 minutes incubation at 80°C the endolysin has almost lost its full activity.

These results emphasize the strong thermal stability of unmodified PVP-SE1gp146, making it an interesting candidate for usage in a hurdle approach for food preservation. Mild heat treatment in combination with PVP-SE1gp146 might be used to efficiently reduce food pathogens without loss of quality of the food product.

References cited

Cheng, X., Zhang, X., Pflugrath, J.W. and Studier, F.W. (1994) The structure of bacteriophage T7 lysozyme, a zinc amidase and an inhibitor of T7 RNA polymerase. Proc Natl Acad Sci U S A 91, 4034-4038.

Briers Y., Lavigne R., Volckaert, G. and Hertveldt, K. (2007a) A standardized approach for accurate quantification of murein hydrolase activity in high-throughput assays. Journal of Biochemical and Biophysical Methods 70, 531-533.

## Claims

1. A polypeptide having endolysin activity comprising the amino acid sequence according to SEQ ID NO: 1 or a fragment or derivative thereof, wherein the fragment comprises the amino acid sequence according to SEQ ID NO: 3 and/or 5, and the derivative has a deletion of 1, 2, 3, 4, 5 or more amino acid residues, an addition of 1, 2, 3, 4, 5 or more amino acid residues, an insertion of 1, 2, 3, 4, 5 or more amino acid residues, or a substitution of an amino acid residue located at a certain position for a different one in the amino acid sequence according to SEQ ID NO: 1, 3, and/or 5, and wherein the fragment and/or the derivative exhibit the lytic activity of the PVPSE1gp146 set forth in

SEQ ID NO:1.

2. The polypeptide according to claim 1, wherein the polypeptide is fused at the N- or C-terminus to a peptide stretch having membrane or LPS disrupting activity.

3. The polypeptide according to claim 1 or 2 comprising additionally a tag, preferably a His6-tag.

4. The polypeptide according to claim 3, wherein said polypeptide comprises the amino acid sequence according to SEQ ID NO:7

5. A fusion protein comprising a polypeptide according to any one of the preceding claims and a peptide stretch fused to said polypeptide at the N- or C-terminus, wherein said peptide stretch is a cationic peptide, polycationic peptide, amphipathic peptide, sushi peptide, defensin, hydrophobic peptide and/or an antimicrobial peptide.

6. The fusion protein according to claim 5, wherein said peptide stretch comprises 5 to 100 amino acid residues, in particular 5 to 50 amino acid residues, in particular 5 to 30 amino acid residues.

7. The fusion protein according to claim 5 or 6, wherein said cationic and/or polycationic peptide stretch comprises at least one amino acid residue selected out of the group consisting of arginine, histidine and lysine residues, in particular wherein at least 70% of the amino acid residues comprised in said peptide stretch are arginine, histidine and/or lysine residues, in particular arginine, and/or lysine residues.

8. The fusion protein according to 5, wherein the amphipathic peptide comprises at least one positively charged amino acid residues selected out of the group consisting of lysine, arginine and histidine residues, combined to at least one hydrophobic amino acid residue selected out of the group consisting of valine, isoleucine, leucine, methionine, phenylalanine, tryptophan, cysteine, alanine, tyrosine, histidine, threonin, serine, proline and glycine residues, in particular wherein at least 70% of the said amino acid residues in said amphipathic peptide are either arginine or lysine residues and at least 30% of the said amino acid residues in said amphipathic peptide are valine, isoleucine, leucine, methionine, phenylalanine, tryptophan, cysteine, alanine, tyrosine, histidine, threonine, serine, proline or glycine residues.

9. The fusion protein according to claim 5, wherein the peptide stretch comprises an amino acid sequence according to SEQ NO:14-19.

10. The fusion protein according to claim 5, wherein the fusion protein comprises the amino acid sequence according to SEQ ID NO:13.

11. An isolated nucleic acid molecule encoding a polypeptide according to any one of claims 1 to 4 or a fusion protein according to any one of the claims 5 to 10.

12. A vector comprising the nucleic acid molecule according to claim 11.

13. A host cell comprising the nucleic acid molecule according to claim 11 or the vector according to claim 12.

14. The polypeptide according to any one of claims 1 to 4 or the fusion protein according to any one of the claims 5 to 10 for use in a method of treatment of the human or animal body by surgery or therapy or for use in diagnostic methods practised on the human or animal body, or for use as an antimicrobial in food or on cosmetics, as disinfecting agent or in the environmental field, or for use in a method for treatment or prevention of Gram-negative bacterial infections.

15. The use of the polypeptide according to any one of claims 1 to 4 or the fusion protein according to any one of the claims 5 to 10 for the treatment or prevention of Gram-negative bacterial contamination of foodstuff, of food processing equipment, of food processing plants, of shelve surfaces coming into contact with foodstuff, of surfaces of food deposit areas, of medical devices, of surfaces in hospitals and surgeries.

16. Pharmaceutical composition comprising the polypeptide according to any one of claims 1 to 4 or the fusion protein according to any one of the claims 5 to 10.

## Patentansprüche

1. Ein Polypeptid mit Endolysinaktivität umfassend die Aminosäuresequenz gemäß SEQ ID NR: 1 oder ein Fragment oder Derivat davon, wobei das Fragment die Aminosäuresequenz gemäß SEQ ID NR: 3 und/oder 5 umfasst, und das Derivat in der Aminosäuresequenz gemäß SEQ ID NR: 1, 3, und/oder 5 eine Deletion von 1, 2, 3, 4, 5 oder mehr Aminosäureresten, eine Addition von 1, 2, 3, 4, 5 oder mehr Aminosäureresten, eine Insertion von 1, 2, 3, 4, 5 oder mehr Aminosäureresten, oder eine Substitution eines sich an einer bestimmten Stelle befindenden Aminosäurerests durch einen anderen hat, und wobei das Fragment und/oder das Derivat die lytische Aktivität des in SEQ ID NR: 1 dargelegten PVPSE1gp146 aufweist.

2. Das Polypeptid gemäß Anspruch 1, wobei das Polypeptid am N- oder C-Terminus mit einen Peptidabschnitt fusioniert ist, der Membran- oder LPS-zerreißende Aktivität hat.

3. Das Polypeptide gemäß Anspruch 1 oder 2, zusätzlich umfassend einen Markierung, vorzugsweise eine $His_6$-Markierung.

4. Das Polypeptide gemäß Anspruch 3, wobei das Polypeptid die Aminosäuresequenz gemäß SEQ ID NR: 7 umfasst.

5. Ein Fusionsprotein umfassend ein Polypeptid gemäß einem der vorgehenden Ansprüche und einen an den N- oder C- Terminus des Polypeptids fusionierten Peptidabschnitt, wobei der Peptidabschnitt ein kationisches Peptid, polykationisches Peptid, amphipathisches Peptid, Sushi-Peptid, Defensin, hydrophobes Peptid und/oder ein antimikrobielles Peptid ist.

6. Das Fusionsprotein gemäß Anspruch 5, wobei der Peptidabschnitt 5 bis 100 Aminosäurereste, insbesondere 5 bis 50 Aminosäurereste, insbesondere 5 bis 30 Aminosäurereste umfasst.

7. Das Fusionsprotein gemäß Anspruch 5 oder 6, wobei der kationische und/oder polykationische Peptidabschnitt mindestens einen Aminosäurerest umfasst, der aus der Gruppe bestehend aus Arginin-, Histidin- und Lysin-Resten ausgewählt ist, wobei insbesondere mindestens 70% der Aminosäurereste, die von dem Peptidabschnitt umfasst sind, Arginin-, Histidin- und/oder Lysin-Reste sind, insbesondere Arginin-, und/oder Lysin-Reste.

8. Das Fusionsprotein gemäß Anspruch 5, wobei das amphipathische Peptid mindestens einen positiv geladen Aminosäurerest, der aus der Gruppe bestehend aus Lysin-, Arginin- und Histidin-Resten ausgewählt ist, kombiniert mit mindestens einem hydrophoben Aminosäurerest ausgewählt aus der Gruppe bestehend aus Valin-, Isoleucin-, Leucin-, Methionin-, Phenylalanin-, Tryptophan-, Cystein-, Alanin-, Tyrosin-, Histidin-, Threonin-, Serin-, Prolin- und Glycin-Resten umfasst, insbesondere wobei mindestens 70% der Aminosäurereste in dem amphipathischen Peptid entweder Arginin- oder Lysin-Reste sind und mindestens 30% der Aminosäurereste in dem amphipathischen Peptid Valin-, Isoleucin-, Leucin-, Methionin-, Phenylalanin-, Tryptophan-, Cystein-, Alanin-, Tyrosin-, Histidin-, Threonin-, Serin-, Prolin- oder Glycin-Reste sind.

9. Das Fusionsprotein gemäß Anspruch 5, wobei der Peptidabschnitt eine Aminosäuresequenz gemäß SEQ ID NR: 14-19 umfasst.

10. Das Fusionsprotein gemäß Anspruch 5, wobei das Fusionsprotein die Aminosäuresequenz gemäß SEQ ID NR: 13 umfasst.

11. Ein isoliertes Nukleinsäuremolekül, das für ein Polypeptid gemäß einen der Ansprüche 1 bis 4 oder für ein Fusionsprotein gemäß einem der Ansprüche 5 bis 10 kodiert.

12. Ein Vektor umfassend das Nukleinsäuremolekül gemäß Anspruch 11.

13. Eine Wirtszelle umfassend das Nukleinsäuremolekül gemäß Anspruch 11 oder den Vektor gemäß Anspruch 12.

14. Das Polypeptid gemäß einem der Ansprüche 1 bis 4 oder das Fusionsprotein gemäß einem der Ansprüche 5 bis 10 zur Verwendung in einem Verfahren zur Behandlung des menschlichen oder tierischen Körpers mittels chirurgischer oder therapeutischer Behandlung oder zur Verwendung in Diagnostiziemerfahren, die am menschlichen oder tierischen Körper vorgenommen werden, oder zur Verwendung als Antimikrobiotikum in Lebensmitteln oder in Kosmetika, als Desinfektionsmittel oder im Bereich der Umwelt, oder zur Verwendung in einem Verfahren zur

Behandlung oder Prävention Gram-negativer bakterieller Infektionen.

**15.** Die Verwendung des Polypeptids gemäß einem der Ansprüche 1 bis 4 oder des Fusionsproteins gemäß einem der Ansprüche 5 bis 10 zur Behandlung oder Prävention Gram-negativer bakterieller Kontamination von Lebensmitteln, von lebensmittelverarbeitendem Gerät, von lebensmittelverarbeitenden Fabriken, von Regaloberflächen, die mit Lebensmitteln in Kontakt kommen, von Oberflächen von Lebensmittellagerungsbereichen, von medizinischen Gerätschaften, von Oberflächen in Krankenhäusern und Operationssälen.

**16.** Pharmazeutische Zusammensetzung umfassend das Polypeptid nach einem der Ansprüche 1 bis 4 oder das Fusionsprotein gemäß einem der Ansprüche 5 bis 10.

## Revendications

**1.** Polypeptide possédant une activité d'endolysine comprenant la séquence d'acides aminés selon SEQ ID NO : 1 ou un fragment ou un dérivé de celle-ci, où le fragment comprend la séquence d'acides aminés selon SEQ ID NO : 3 et/ou 5, et le dérivé comporte une délétion de 1, 2, 3, 4, 5 résidus d'acides aminés ou plus, une addition de 1, 2, 3, 4, 5 résidus d'acides aminés ou plus, une insertion de 1, 2, 3, 4, 5 résidus d'acides aminés ou plus, ou une substitution d'un résidu d'acide aminé localisé au niveau d'une certaine position à un résidu d'acide aminé différent dans la séquence d'acides aminés selon SEQ ID NO : 1, 3 et/ou 5, et où le fragment et/ou le dérivé présente l'activité lytique de la PVPSE1gp146 représentée par SEQ ID NO : 1.

**2.** Polypeptide selon la revendication 1, où le polypeptide est fusionné à l'extrémité N- ou C-terminale à une extension peptidique possédant une activité de rupture de la membrane ou du LPS.

**3.** Polypeptide selon la revendication 1 ou 2, comprenant en outre un marqueur, de préférence un marqueur His6.

**4.** Polypeptide selon la revendication 3, où ledit polypeptide comprend la séquence d'acides aminés selon SEQ ID NO : 7.

**5.** Protéine de fusion comprenant un polypeptide selon l'une quelconque des revendications précédentes et une extension peptidique fusionnée audit polypeptide à l'extrémité N- ou C-terminale, où ladite extension peptidique est un peptide cationique, un peptide polycationique, un peptide amphipathique, un peptide sushi, une défensine, un peptide hydrophobe et/ou un peptide antimicrobien.

**6.** Protéine de fusion selon la revendication 5, où ladite extension peptidique comprend 5 à 100 résidus d'acides aminés, en particulier 5 à 50 résidus d'acides aminés, en particulier 5 à 30 résidus d'acides aminés.

**7.** Protéine de fusion selon la revendication 5 ou 6, où ladite extension peptidique cationique et/ou polycationique comprend au moins un résidu d'acide aminé choisi dans le groupe constitué des résidus d'arginine, d'histidine et de lysine, en particulier où au moins 70 % des résidus d'acides aminés compris dans ladite extension peptidique sont des résidus d'arginine, d'histidine et/ou de lysine, en particulier des résidus d'arginine et/ou de lysine.

**8.** Protéine de fusion selon la revendication 5, où le peptide amphipathique comprend au moins un résidu d'acide aminé chargé positivement choisi dans le groupe constitué des résidus de lysine, d'arginine et d'histidine, combiné à au moins un résidu d'acide aminé hydrophobe choisi dans le groupe constitué des résidus de valine, d'isoleucine, de leucine, de méthionine, de phénylalanine, de tryptophane, de cystéine, d'alanine, de tyrosine, d'histidine, de thréonine, de sérine, de proline et de glycine, en particulier où au moins 70 % desdits résidus d'acides aminés dans ledit peptide amphipathique sont des résidus soit d'arginine soit de lysine et au moins 30 % desdits résidus d'acides aminés dans ledit peptide amphipathique sont des résidus de valine, d'isoleucine, de leucine, de méthionine, de phénylalanine, de tryptophane, de cystéine, d'alanine, de tyrosine, d'histidine, de thréonine, de sérine, de proline ou de glycine.

**9.** Protéine de fusion selon la revendication 5, où l'extension peptidique comprend une séquence d'acides aminés selon SEQ ID NO: 14 à 19.

**10.** Protéine de fusion selon la revendication 5, où la protéine de fusion comprend la séquence d'acides aminés selon SEQ ID NO : 13.

11. Molécule d'acide nucléique isolée codant pour un polypeptide selon l'une quelconque des revendications 1 à 4 ou une protéine de fusion selon l'une quelconque des revendications 5 à 10.

12. Vecteur comprenant la molécule d'acide nucléique selon la revendication 11.

13. Cellule hôte comprenant la molécule d'acide nucléique selon la revendication 11 ou le vecteur selon la revendication 12.

14. Polypeptide selon l'une quelconque des revendications 1 à 4 ou protéine de fusion selon l'une quelconque des revendications 5 à 10 pour une utilisation dans une méthode de traitement du corps humain ou animal par chirurgie ou thérapie ou pour une utilisation dans des méthodes de diagnostic pratiquées sur le corps humain ou animal, ou pour une utilisation en tant qu'antimicrobien dans des aliments ou sur des produits cosmétiques, en tant qu'agent désinfectant ou dans le domaine environnemental, ou pour une utilisation dans une méthode de traitement ou de prévention d'infections à bactéries Gram négatif.

15. Utilisation du polypeptide selon l'une quelconque des revendications 1 à 4 ou de la protéine de fusion selon l'une quelconque des revendications 5 à 10 pour le traitement ou la prévention d'une contamination avec des bactéries Gram négatif d'aliments, ou d'un équipement de traitement d'aliments, ou d'usines de traitement d'aliments, ou de surfaces d'étagères entrant en contact avec des aliments, de surfaces de zones de stockage d'aliments, de dispositifs médicaux, de surfaces dans des hôpitaux et des blocs chirurgicaux.

16. Composition pharmaceutique comprenant le polypeptide selon l'une quelconque des revendications 1 à 4 ou la protéine de fusion selon l'une quelconque des revendications 5 à 10.

## FIGURES

**> DNA sequence of wt ORF146 of PVPSE1 (711 bp)**

ATGAATGCTGCAATTGCGGAGATTCAGCGTATGCTGATCGAAGGTGGGTTTAGCGTCG
GCAAGTCTGGTGCTGATGGATTGTACGGACCCGCTACAAAAGCCGCACTGCAAAAGTG
CATTGCACAGGCTACCAGTGGAAACAATAAAGGAGGTACTTTGAAACTCACCCAAGCAC
AACTGGACAAAATCTTCCCCGTTGGTGCAAGTTCTGGGAGGAATGCAAAATTCCTGAAG
CCGCTCAATGACCTGTTTGAAAAGACAGAGATTAATACGGTAAATCGGGTTGCAGGATT
CCTGTCTCAGATTGGTGTGGAGTCGGCGGAGTTCCGGTATGTACGTGAACTCGGTAAC
GATGCCTACTTTGACAAGTACGACACTGGTCCTATTGCAGAAAGACTTGGAAACACACC
CCAGAAAGATGGGGATGGTGCCAAGTACAAGGGGAGAGGTCTGATTCAGGTGACCGGA
CTCGCAAACTACAAGGCTTGCGGTAAAGCACTCGGTCTTGACCTCGTTAACCACCCTGA
GTTGCTTGAACAGCCTGAGTATGCAGTTGCCAGCGCTGGTTGGTATTGGGACACGAGA
AACATCAACGCCGCTTGCGATGCTGATGATATCGTGAAAATTACCAAGCTGGTAAACGG
TGGTACAAATCACCTTGCCGAGCGCACAGCCTATTACAAAAAGGCAAAATCTGTTCTAA
CCTCGTAA

**> Protein sequence of wt PVPSE1gp146 (236 amino acids)**

MNAAIAEIQRMLIEGGFSVGKSGADGLYGPATKAALQKCIAQATSGNNKGGTLKLTQAQLDK
IFPVGASSGRNAKLKPLNDLFEKTEINTVNRVAGFLSQIGVESAEFRYVRELGNDAYFDKYD
TGPIAERLGNTPQKDGDGAKYKGRGLIQVTGLANYKACGKALGLDLVNHPELLEQPEYAVA
SAGWYWDTRNINAACDADDIVKITKLVNGGTNHLAERTAYYKKAKSVLTS

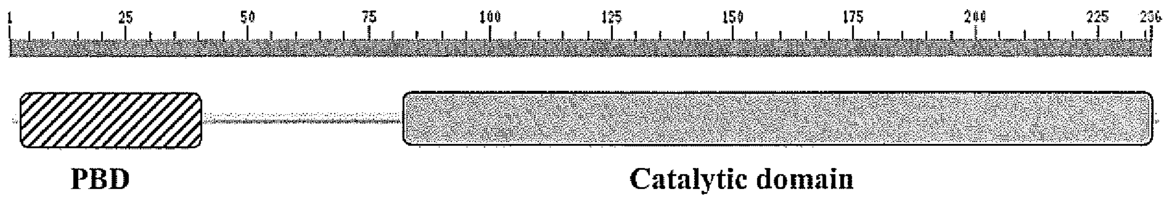


Figure 1

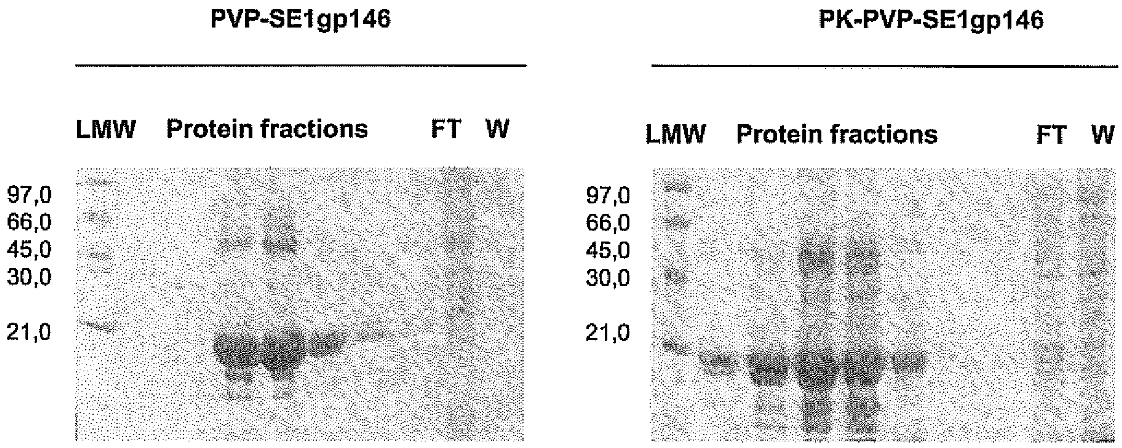
PVP-SE1gp146
LMW
Protein fractions
FT
W
97,0
66,0
45,0
30,0
21,0
PK-PVP-SE1gp146
LMW
Protein fractions
FT
W
97,0
66,0
45,0
30,0
21,0

Fig. 2

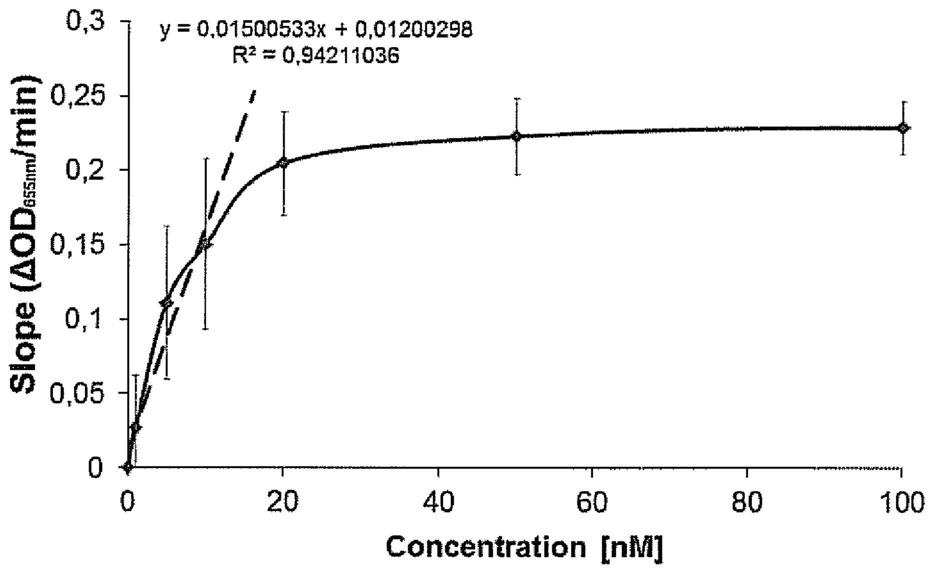
y = 0,01500533x + 0,01200298
R² = 0,94211036
Slope (ΔOD655nm/min)
0,3
0,25
0,2
0,15
0,1
0,05
0
0
20
40
60
80
100
Concentration [nM]

Fig. 3

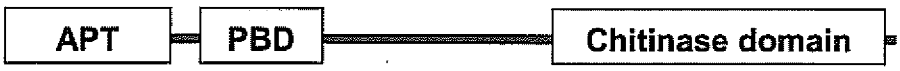


Fig. 4

A

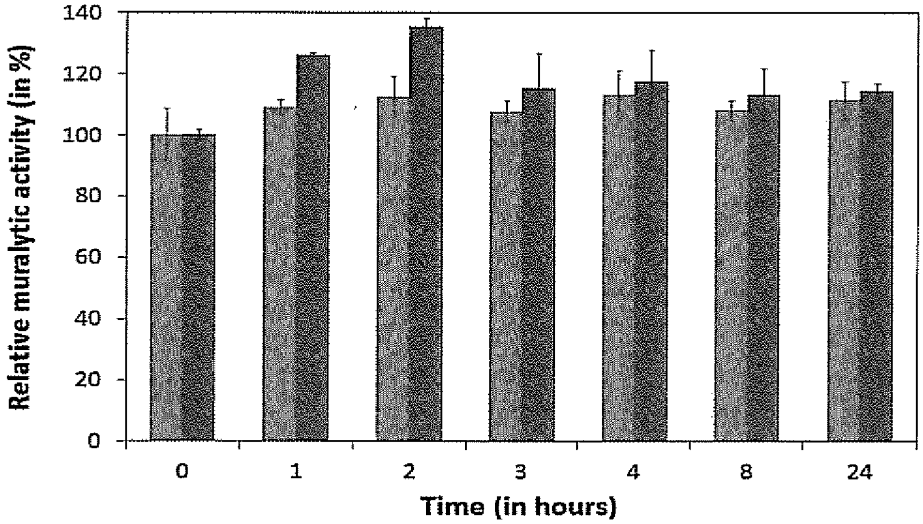
Relative muralytic activity (in %)
140
120
100
80
60
40
20
0
0
1
2
3
4
8
24
Time (in hours)

B

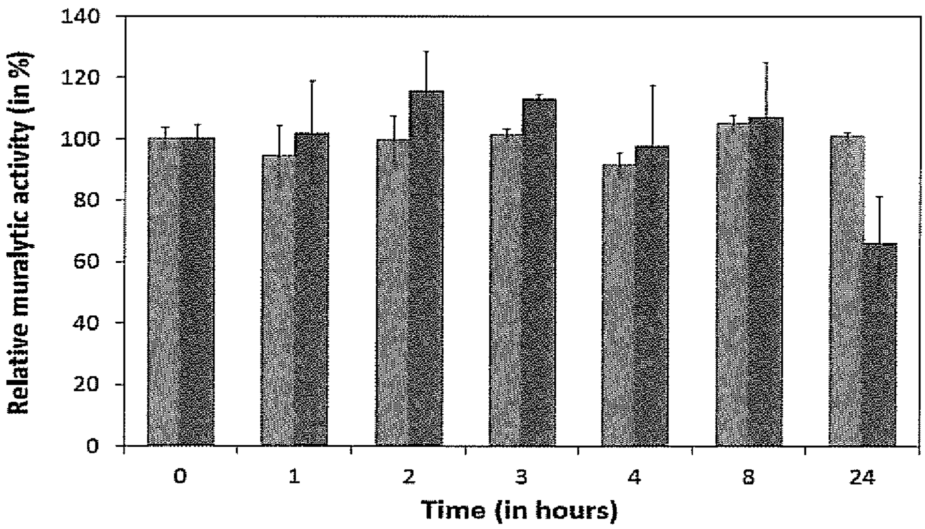
Relative muralytic activity (in %)
140
120
100
80
60
40
20
0
0
1
2
3
4
8
24
Time (in hours)

Fig. 5

A

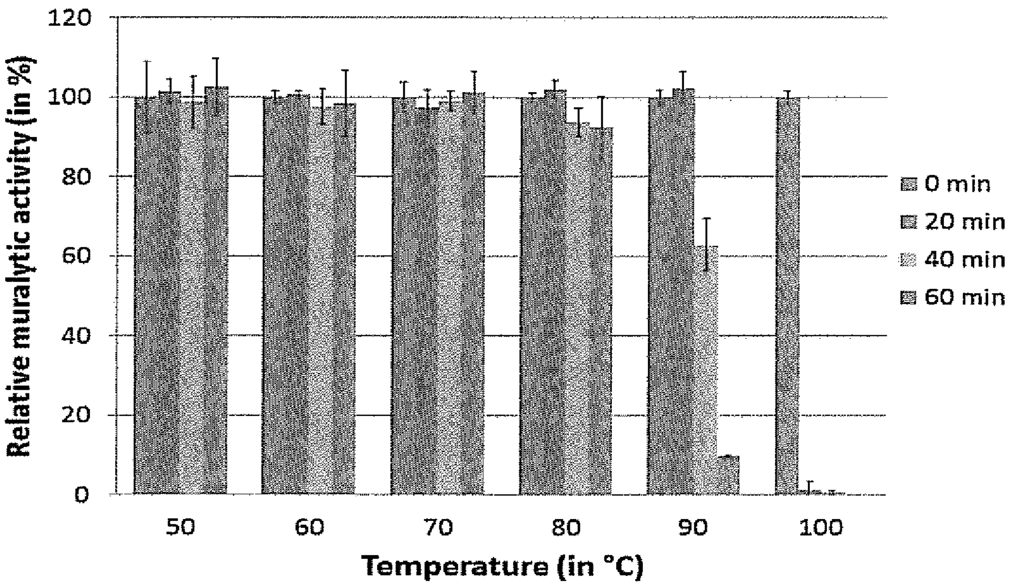
Relative muralytic activity (in %)
120
100
80
60
40
20
0
50
60
70
80
90
100
Temperature (in °C)
0 min
20 min
40 min
60 min

B

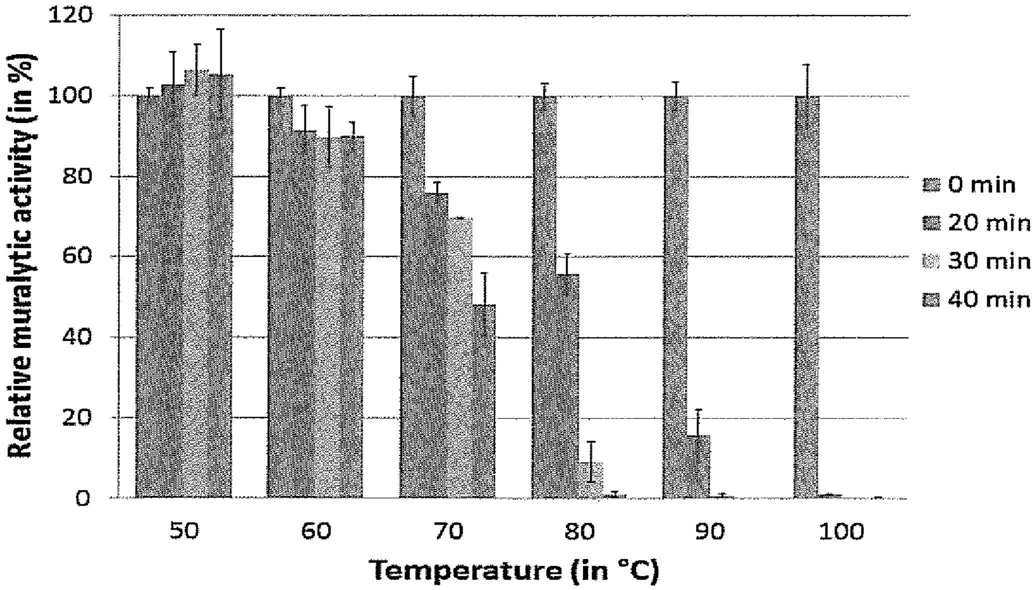
Relative muralytic activity (in %)
120
100
80
60
40
20
0
50
60
70
80
90
100
Temperature (in °C)
0 min
20 min
30 min
40 min

Fig. 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- GB 2243611 A, Gasson
- WO 2010023207 A
- GB 1018518 A


### Non-patent literature cited in the description


- **MELO et al.** *Nature reviews, Microbiology,* 2009, 245
- **SANTOS et al.** *Appl Environ Microbiol.,* November 2010, vol. 76 (21), 7338-42
- **SAMBROOK, J. et al.** Molecular Cloning: A Laboratory Manual. Cold Spring Harbor Press, 1989
- **BAE et al.** *Bioinformatics,* 2005, vol. 21, 2264-2270
- **GEORGE ; HERINGA.** *Protein Engineering,* 2003, vol. 15, 871-879
- **BRIERS et al.** *J. Biochem. Biophys Methods,* 2007, vol. 70, 531-533
- **VAARA M.** Agents that increase the permeability of the outer membrane. *Microbiol Rev.,* September 1992, vol. 56 (3), 395-441
- **SAMBROCK et al.** Molecular Cloning: A Laboratory Manual. Cold Spring Harbor Laboratory Press, 1989
- **CHENG, X. ; ZHANG, X. ; PFLUGRATH, J.W. ; STUDIER, F.W.** The structure of bacteriophage T7 lysozyme, a zinc amidase and an inhibitor of T7 RNA polymerase. *Proc Natl Acad Sci U S A,* 1994, vol. 91, 4034-4038
- **BRIERS Y. ; LAVIGNE R. ; VOLCKAERT, G. ; HERTVELDT, K.** A standardized approach for accurate quantification of murein hydrolase activity in high-throughput assays. *Journal of Biochemical and Biophysical Methods,* 2007, vol. 70, 531-533